# EUROPEAN PATENT SPECIFICATION

(11) **EP 2 482 812 B1**
(45) Date of publication and mention of the grant of the patent: **11.01.2023**
(21) Application number: 10760351.6
(22) Date of filing: 01.10.2010
(51) Int. Cl.: A61K 31/155, A61K 31/522, A61P 3/00, A61K 47/18

(54) **PHARMACEUTICAL COMPOSITIONS COMPRISING BI-1356 AND METFORMIN**
PHARMAZEUTISCHE ZUSAMMENSETZUNGEN ENTHALTEND BI-1356 UND METFORMIN
COMPOSITIONS PHARMACEUTIQUES CONTENANT LE BI-1356 ET LA METFORMINE

(30) Priority: 02.10.2009 EP 09172117
(43) Date of publication of application: 08.08.2012
(73) Proprietor: Boehringer Ingelheim International GmbH, 55216 Ingelheim am Rhein (DE)
(72) Inventor: MEINICKE, Thomas, 55216 Ingelheim Am Rhein (DE)
(74) Representative: Simon, Elke Anna Maria
(86) International application number: PCT/EP2010/064691
(87) International publication number: WO 2011/039367

(56) References cited:
- WO-A2-2007/078726
- WO-A2-2007/128761
- WO-A2-2009/121945
- Boehringer Ingelheim Pharmaceuticals: "Safety and Efficacy of BI 1356 Plus Metformin in Type 2 Diabetes, Factorial Design", , 24 November 2008 (2008-11-24), XP002614334, Retrieved from the Internet: URL:http://clinicaltrials.gov/archive/NCT0 0798161/2008_11_24 [retrieved on 2010-12-14]
- ANONYMOUS: "efficacy and safety of BI 1356 in combination with metformin in patietns with type 2 diabetes", INTERNET CITATION, 13 February 2008 (2008-02-13), pages 1-5, XP002495205, Retrieved from the Internet: URL:http://clinicaltrial.gov/ct2/show/NCT0 0622284 [retrieved on 2008-09-09]
- "EFFICANCY AND SAFETY OF BI 1356 VS. PLACEBO ADDED TO METFORMIN BACKGROUND THERAPY IN PATIENTS WITH TYPE 2 DIABETES", Clinical trials INTERNET CITATION, 25 January 2008 (2008-01-25), pages 1-3, XP002517666, Retrieved from the Internet: URL:http://clinicaltrials.gov/archive/NCT0 0601250/2008_01_25 [retrieved on 2009-02-27]
- Boehringer Ingelheim Pharmaceuticals: "BI 1356 in Combination With Metformin and a Sulphonylurea in Type 2 Diabetes", druglib.com , 3 November 2008 (2008-11-03), XP002614335, Retrieved from the Internet: URL:http://65.18.172.42/trial/72/NCT006024 72.html [retrieved on 2010-12-10]
- Merck: "Initial therapy with JANUMET (sitagliptin/metformin)", , 8 June 2009 (2009-06-08), XP002614336, Retrieved from the Internet: URL:http://www.webwire.com/ViewPressRel.as p?aId=96695 [retrieved on 2010-12-13]
- WILLIAMS-HERMAN D ET AL: "Efficacy and safety of initial combination therapy with sitagliptin and metformin in patients with type 2 diabetes: A 54-week study", CURRENT MEDICAL RESEARCH AND OPINION, INFORMA HEALTHCARE, GB, vol. 25, no. 3, 1 January 2009 (2009-01-01), pages 569-583, XP008130502, ISSN: 0300-7995, DOI: DOI:10.1185/03007990802705679
- THOMAS L ET AL: "BI 1356, a novel and selective xanthine based DPP-IV inhibitor, exhibits a superior profile when compared to sitagliptin and vildagliptin", DIABETOLOGIA, vol. 50, no. Suppl. 1, September 2007 (2007-09), page S363, XP8130500, & 43RD ANNUAL MEETING OF THE EUROPEAN-ASSOCIATION-FOR-THE-STUDY-OF-DIAB ETES; AMSTERDAM, NETHERLANDS; SEPTEMBER 18 -21, 2007 ISSN: 0012-186X
- AHREN B: "DPP-4 inhibitors", BEST PRACTICE AND RESEARCH IN CLINICAL ENDOCRINOLOGY AND METABOLISM - NEW THERAPIES FOR DIABETES 200712 GB LNKD- DOI:10.1016/J.BEEM.2007.07.005, vol. 21, no. 4, December 2007 (2007-12), pages 517-533, XP022381062, ISSN: 1521-690X
- Boehringer Ingelheim Pharmaceuticals: "Efficacy and Safety of BI 1356 BS in Combination With Metformin in Patients With type2 Diabetes", , 27 January 2009 (2009-01-27), XP002624499, Retrieved from the Internet: URL:http://clinicaltrials.gov/ct2/show/NCT 00309608 [retrieved on 2010-12-08]

## Description

The present invention relates to a pharmaceutical composition comprising or made from a DPP-4 inhibitor which is 1-[(4-methyl-quinazolin-2-yl)methyl]-3-methyl-7-(2-butyn-1-yl)-8-(3-(R)-amino-piperidin-1-yl)-xanthine,
metformin,
a stabilizer which is a basic amino acid,
and one or more pharmaceutical excipients;
for use in treating and/or preventing (including slowing the progression and/or delaying the onset) of metabolic diseases, especially type 2 diabetes mellitus and conditions related thereto (e.g. diabetic complications),
either in type 2 diabetes patients who have not been previously treated with an antihyperglycemic agent,
or in type 2 diabetes patients with insufficient glycemic control despite therapy with one or two conventional antihyperglycemic agents selected from metformin, sulphonylureas, thiazolidinediones (e.g. pioglitazone), glinides, alpha-glucosidase blockers, GLP-1 or GLP-1 analogues, and insulin or insulin analogues;
optionally in combination with one or more other active substances.

The present invention further relates to a pharmaceutical composition comprising or made from
a DPP-4 inhibitor which is 1-[(4-methyl-quinazolin-2-yl)methyl]-3-methyl-7-(2-butyn-1-yl)-8-(3-(R)-amino-piperidin-1-yl)-xanthine,
metformin,
a stabilizer which is a basic amino acid,
and one or more pharmaceutical excipients;
for use in one or more of the following methods:
   - preventing, slowing the progression of, delaying the onset of or treating a metabolic disorder or disease, such as e.g. type 1 diabetes mellitus, type 2 diabetes mellitus, impaired glucose tolerance (IGT), impaired fasting blood glucose (IFG), hyperglycemia, postprandial hyperglycemia, overweight, obesity, dyslipidemia, hyperlipidemia, hypercholesterolemia, hypertension, atherosclerosis, endothelial dysfunction, osteoporosis, chronic systemic inflammation, non-alcoholic fatty liver disease (NAFLD), retinopathy, neuropathy, nephropathy and/or metabolic syndrome;
   - improving glycemic control and/or for reducing of fasting plasma glucose, of postprandial plasma glucose and/or of glycosylated hemoglobin HbA1c;
   - preventing, slowing, delaying the onset of or reversing progression from impaired glucose tolerance (IGT), impaired fasting blood glucose (IFG), insulin resistance and/or from metabolic syndrome to type 2 diabetes mellitus;
   - preventing, reducing the risk of, slowing the progression of, delaying the onset of or treating of complications of diabetes mellitus such as micro- and macrovascular diseases, such as nephropathy, micro- or macroalbuminuria, proteinuria, retinopathy, cataracts, neuropathy, learning or memory impairment, neurodegenerative or cognitive disorders, cardio- or cerebrovascular diseases, tissue ischaemia, diabetic foot or ulcus, atherosclerosis, hypertension, endothelial dysfunction, myocardial infarction, acute coronary syndrome, unstable angina pectoris, stable angina pectoris, peripheral arterial occlusive disease, cardiomyopathy, heart failure, heart rhythm disorders, vascular restenosis, and/or stroke;
   - reducing body weight or preventing an increase in body weight or facilitating a reduction in body weight;
   - preventing, slowing the progression of, delaying the onset of or treating the degeneration of pancreatic beta cells and/or the decline of the functionality of pancreatic beta cells and/or for improving and/or restoring the functionality of pancreatic beta cells and/or stimulating and/or restoring the functionality of pancreatic insulin secretion;
   - preventing, slowing the progression of, delaying the onset of or treating non alcoholic fatty liver disease (NAFLD) including hepatic steatosis, non-alcoholic steatohepatitis (NASH) and/or liver fibrosis;
   - preventing, slowing the progression of, delaying the onset of or treating type 2 diabetes with primary or secondary failure to conventional (oral or non-oral) antihyperglycemic mono- or combination therapy;
   - achieving a reduction in the dose of conventional antihyperglycemic medication required for adequate therapeutic effect;
   - reducing the risk for adverse effects associated with conventional antihyperglycemic medication; and/or
   - maintaining and/or improving the insulin sensitivity and/or for treating or preventing hyperinsulinemia and/or insulin resistance;
either in a type 2 diabetes patient who has not been previously treated with an antihyperglycemic agent or in a type 2 diabetes patient with insufficient glycemic control despite therapy with one or two conventional antihyperglycemic agents selected from metformin, sulphonylureas, thiazolidinediones, glinides, alpha-glucosidase blockers, GLP-1 or GLP-1 analogues, and insulin or insulin analogues;
optionally in combination with one or more other active substances.

The present invention further relates to the pharmaceutical composition for use as mentioned afore, which is a solid pharmaceutical composition (e.g. oral tablet) comprising or made from 1-[(4-methyl-quinazolin-2-yl)methyl]-3-methyl-7-(2-butyn-1-yl)-8-(3-(R)-amino-piperidin-1-yl)-xanthine,
metformin hydrochloride,
L-arginine (particularly as stabilizer),
and one or more fillers, one or more binders, one or more glidants and/or one or more lubricants.

Reference is made to the present claims for further embodiments of the present invention.

The scope of the invention is defined by the claims.

The references to methods of medical treatment in the following description are to be interpreted as references to the compounds, pharmaceutical compositions, combinations, medicaments etc. of the present invention for use in those methods.

The present disclosure relates to certain therapeutic uses of a combination comprising a certain DPP-4 inhibitor and metformin, such as e.g. for treating and/or preventing metabolic diseases, especially type 2 diabetes mellitus and/or conditions related thereto (e.g. diabetic complications).

Type 2 diabetes mellitus is a common chronic and progressive disease arising from a complex pathophysiology involving the dual endocrine effects of insulin resistance and impaired insulin secretion. The treatment of type 2 diabetes typically begins with diet and exercise, followed by oral antidiabetic monotherapy, and although conventional monotherapy may initially control blood glucose in some patients, it is however associated with a high secondary failure rate. The limitations of single-agent therapy for maintaining glycemic control may be overcome, at least in some patients, and for a limited period of time by combining multiple drugs to achieve reductions in blood glucose that cannot be sustained during long-term therapy with single agents. Available data support the conclusion that in most patients with type 2 diabetes current monotherapy will fail and treatment with multiple drugs will be required.

But, because type 2 diabetes is a progressive disease, even patients with good initial responses to conventional combination therapy will eventually require an increase of the dosage or further treatment with insulin because the blood glucose level is very difficult to maintain stable for a long period of time. Although existing combination therapy has the potential to enhance glycemic control, it is not without limitations (especially with regard to long term efficacy). Further, the traditional combinations have shown an increased risk for side effects, such as hypoglycemia or weight gain.

Thus, for many patients, these existing drug therapies result in progressive deterioriation in glycemic control despite treatment and do not sufficiently control glycemia especially over long-term and thus fail to achieve and to maintain metabolic control in advanced or late stage type 2 diabetes, including diabetes with inadequate glycemic control despite conventional oral or non-oral antidiabetic medication.

Therefore, although intensive treatment of hyperglycemia can reduce the incidence of chronic damages, many patients with type 2 diabetes remain inadequately treated, partly because of limitations in long term efficacy, tolerability and dosing inconvenience of conventional antihyperglycemic therapies, patients' poor adherence or comorbidities.

This high incidence of therapeutic failure is a major contributor to the high rate of long-term hyperglycemia-associated complications or chronic damages (including micro- and makrovascular complications such as e.g. diabetic nephrophathy, retinopathy or neuropathy, or cardiovascular or cerebrovascular complications such as myocardial infarction, stroke or death) in patients with type 2 diabetes.

Oral antidiabetic drugs conventionally used in therapy (such as e.g. first- or second-line, and/or mono- or (initial or add-on) combination therapy) include, without being restricted thereto, metformin, sulphonylureas, thiazolidinediones, glinides and α-glucosidase inhibitors.

Non-oral antidiabetic drugs conventionally used in therapy (such as e.g. first- or second-line, and/or mono- or (initial or add-on) combination therapy) include, without being restricted thereto, GLP-1 or GLP-1 analogues, and insulin or insulin analogues.

However, the use of these conventional antidiabetic or antihyperglycemic agents can be associated with various adverse effects. For example, metformin can be associated with lactic acidosis or gastrointestinal side effects; sulfonylureas, glinides and insulin or insulin analogues can be associated with hypoglycemia and weight gain; thiazolidinediones can be associated with edema, bone fracture, weight gain and heart failure/cardiac effects; and alpha-glucosidase blockers and GLP-1 or GLP-1 analogues can be associated with gastrointestinal adverse effects (e.g. dyspepsia, flatulence or diarrhea, or nausea or vomiting).

Therefore, it remains a need in the art to provide efficacious, safe and tolerable antidiabetic therapies both for patients who have not previously been treated with an antidiabetic drug (drug-naïve patients) and for patients with advanced or late stage type 2 diabetes mellitus, including patients with inadequate glycemic control on conventional oral and/or non-oral antidiabetic drugs, such as e.g. metformin, sulphonylureas, thiazolidinediones, glinides and/or α-glucosidase inhibitors, and/or GLP-1 or GLP-1 analogues, and/or insulin or insulin analogues.

Further, within the therapy of type 2 diabetes it is a need for treating the condition effectively, avoiding the complications inherent to the condition, and delaying disease progression.

Furthermore, it remains a need that antidiabetic treatments not only prevent the long-term complications often found in advanced stages of diabetes disease, but also are a therapeutic option in those diabetes patients who have developed complications, such as renal impairment.

Moreover, it remains a need to provide prevention or reduction of risk for adverse effects associated with conventional antidiabetic therapies.

The following internet references describe clinical trials in which BI 1356 is said to be given in combination with metformin in patients with type 2 diabetes:
ClinicalTrials.gov Identifier NCT00798161, Safety and Efficacy of Linagliptin (BI 1356) Plus Metformin in Type 2 Diabetes, Factorial Design;
ClinicalTrials.gov Identifier NCT00622284, Efficacy and Safety of BI 1356 in Combination With Metformin in Patients With Type 2 Diabetes;
ClinicalTrials.gov Identifier NCT00601250, Efficacy and Safety of BI 1356 vs. Placebo Added to Metformin Background Therapy in Patients With Type 2 Diabetes;
ClinicalTrials.gov Identifier NCT00602472, BI 1356 in Combination With Metformin and a Sulphonylurea in Type 2 Diabetes.

The reference WO 2004/032909 describes a stabilized pharmaceutical composition in an oral dosage form comprising a cyano-quinolinyl-amide compound, particularly EKB-569 (an EGFR inhibitor), said pharmaceutical composition containing at least one basic excipient and at least one pharmaceutically acceptable excipient.

The reference WO 2007/128761 describes the use of selected DPP-4 inhibitors for treating physiological functional disorders.

The reference WO 2007/078726 describes pharmaceutical compositions comprising fixed-dose combinations of a DPP-4 inhibitor and metformin.

Within the scope of the present disclosure it has now been found that certain DPP-4 inhibitors as defined herein as well as combinations or pharmaceutical compositions according to this disclosure of these DPP-4 inhibitors with metformin have unexpected and particularly advantageous properties, which make them suitable for the purpose of this disclosure and/or for fulfilling one or more of above needs, such as e.g. for improving glycemic control as well as for treating and/or preventing (including slowing the progression or delaying the onset) of metabolic diseases, particularly diabetes (especially type 2 diabetes mellitus) and conditions related thereto (e.g. diabetic complications), in drug naïve type 2 diabetes patients and/or in patients with advanced or late stage type 2 diabetes, including patients with insufficient glycemic control despite a therapy with an oral and/or a non-oral antidiabetic or antihyperglycemic drug and/or with indication on insulin.

The present disclosure thus relates to a combination or a pharmaceutical composition comprising a certain DPP-4 inhibitor (particularly BI 1356) and metformin for simultaneous, separate or sequential use in the therapies described herein.

The present disclosure also relates to a fixed or free combination or pharmaceutical composition comprising or made of
a certain DPP-4 inhibitor ((particularly BI 1356) ) and metformin each as defined herein,
and optionally one or more pharmaceutically acceptable carriers and/or auxiliaries (including excipients, stabilizers or the like), for therapeutic uses as described herein,
such as e.g. for improving glycemic control and/or for use in treating and/or preventing (including slowing the progression and/or delaying the onset) of metabolic diseases,
especially type 2 diabetes mellitus and conditions related thereto (e.g. diabetic complications),
either as first line therapy, i.e. in type 2 diabetes patients who have not previously treated with an antihyperglycemic agent (drug-naive patients),
or as second or third line therapy, i.e. in type 2 diabetes patients with insufficient glycemic control despite therapy with one or two conventional antihyperglycemic agents selected from metformin, sulphonylureas, thiazolidinediones (e.g. pioglitazone), glinides, alpha-glucosidase blockers, GLP-1 or GLP-1 analogues, and insulin or insulin analogues;
optionally in combination with one or more other active substances, such as e.g. any of those mentioned herein,
such as e.g. optionally in combination with one conventional antihyperglycemic agent selected from sulphonylureas, thiazolidinediones (e.g. pioglitazone), glinides, alpha-glucosidase blockers, GLP-1 or GLP-1 analogues, and insulin or insulin analogues.

The present disclosure also relates to therapeutic uses as described herein of a pharmaceutical composition according to this disclosure comprising a fixed dose combination formulation of a DPP-4 inhibitor drug and the partner drug metformin.

In one embodiment, the present disclosure also relates to a fixed or free combination or pharmaceutical composition as described herein before and herein after, optionally in combination with one conventional antihyperglycemic agent selected from sulphonylureas, thiazolidinediones, glinides, alpha-glucosidase blockers, GLP-1 or GLP-1 analogues, and insulin or insulin analogues,
for use in treating and/or preventing (including slowing the progression and/or delaying the onset) of metabolic diseases, especially type 2 diabetes mellitus and conditions related thereto (e.g. diabetic complications),
either as first line therapy, i.e. in type 2 diabetes patients who have not previously treated with an antihyperglycemic agent (drug-naïve patients),
or as second or third line therapy, i.e. in type 2 diabetes patients with insufficient glycemic control despite therapy with one or two conventional antihyperglycemic agents selected from metformin, sulphonylureas, thiazolidinediones, glinides, alpha-glucosidase blockers, GLP-1 or GLP-1 analogues, and insulin or insulin analogues.

In a particular embodiment, the present disclosure relates to a pharmaceutical composition as described herein, for use in treating and/or preventing (including slowing the progression and/or delaying the onset) of metabolic diseases, especially type 2 diabetes mellitus and conditions related thereto (e.g. diabetic complications), in type 2 diabetes patients with insufficient glycemic control despite mono-therapy with metformin.

In another particular embodiment, the present disclosure also relates to a pharmaceutical composition as described herein, in combination with a sulphonylurea, for use in treating and/or preventing (including slowing the progression and/or delaying the onset) of metabolic diseases, especially type 2 diabetes mellitus and conditions related thereto (e.g. diabetic complications), in type 2 diabetes patients with insufficient glycemic control despite dual combination therapy with metformin and a sulphonylurea.

In another particular embodiment, the present disclosure also relates to a pharmaceutical composition as described herein, in combination with a thiazolidinedione (e.g. pioglitazone), for use in treating and/or preventing (including slowing the progression and/or delaying the onset) of metabolic diseases, especially type 2 diabetes mellitus and conditions related thereto (e.g. diabetic complications), in type 2 diabetes patients with insufficient glycemic control despite dual combination therapy with metformin and a thiazolidinedione (e.g. pioglitazone).

In another particular embodiment, the present disclosure also relates to a pharmaceutical composition as described herein, for use in treating and/or preventing (including slowing the progression and/or delaying the onset) of metabolic diseases, especially type 2 diabetes mellitus and conditions related thereto (e.g. diabetic complications), in drug-naïve type 2 diabetes patients (e.g. as first line therapy), such as e.g. as early or initial combination therapy.

The present disclosure further provides the use of a pharmaceutical composition as defined herein for the manufacture of a medicament for treating and/or preventing metabolic diseases, particularly type 2 diabetes mellitus and conditions related thereto (e.g. diabetic complications), e.g. as first, second or third line therapy as described herein.

The present disclosure further provides a pharmaceutical package comprising a pharmaceutical composition as defined herein and optionally instructions for its use, optionally in combination with one or more other active substances, in the treatment and/or prevention of metabolic diseases, particularly type 2 diabetes mellitus and conditions related thereto (e.g. diabetic complications), in drug-naïve patients or in patients with insufficient glycemic control despite therapy with one or two conventional antihyperglycemic agents selected from metformin, sulphonylureas, thiazolidinediones, glinides, alpha-glucosidase blockers, GLP-1 or GLP-1 analogues, and insulin or insulin analogues.

The present disclosure further provides a medicament for use in the treatment and/or prevention of metabolic diseases, particularly type 2 diabetes mellitus and conditions related thereto (e.g. diabetic complications), in drug-naïve patients or in patients with insufficient glycemic control despite therapy with one or two conventional antihyperglycemic agents selected from metformin, sulphonylureas, thiazolidinediones, glinides, alpha-glucosidase blockers, GLP-1 or GLP-1 analogues, and insulin or insulin analogues; said medicament comprising a pharmaceutical composition as defined herein and optionally one or more other active substances, such as e.g. any of those mentioned herein, such as e.g. for separate, sequential, simultaneous, concurrent or chronologically staggered use of the active ingredients.

The present disclosure further provides a method of treating and/or preventing metabolic diseases, particularly type 2 diabetes mellitus and conditions related thereto (e.g. diabetic complications), in drug-naïve patients (e.g. as first line therapy) or in patients with insufficient glycemic control despite therapy with one or two conventional antihyperglycemic agents selected from metformin, sulphonylureas, thiazolidinediones, glinides, alpha-glucosidase blockers, GLP-1 or GLP-1 analogues, and insulin or insulin analogues (e.g. as second or third line therapy); said method comprising administering to a subject in need thereof (particularly a human patient) an effective amount of a pharmaceutically composition as defined herein, optionally alone or in combination, such as e.g. separately, sequentially, simultaneously, concurrently or chronologically staggered, with an effective amount of one or more other active substances, such as e.g. any of those mentioned herein.

The present disclosure further provides the use of a pharmaceutical combination or composition as defined herein before and herein after comprising BI 1356 and metformin, for the manufacture of a medicament for one or more of the following purposes:
- preventing, slowing the progression of, delaying the onset of or treating a metabolic disorder or disease, such as e.g. type 1 diabetes mellitus, type 2 diabetes mellitus, impaired glucose tolerance (IGT), impaired fasting blood glucose (IFG), hyperglycemia, postprandial hyperglycemia, overweight, obesity, dyslipidemia, hyperlipidemia, postprandial hyperlipidemia, hypercholesterolemia, hypertension, atherosclerosis, endothelial dysfunction, osteoporosis, chronic systemic inflammation, non-alcoholic fatty liver disease (NAFLD), retinopathy, neuropathy, nephropathy, polycystic ovarian syndrome and/or metabolic syndrome;
- improving and/or maintaining glycemic control and/or for reducing of fasting plasma glucose, of postprandial plasma glucose and/or of glycosylated hemoglobin HbA1c;
- preventing, slowing, delaying the onset of or reversing progression from impaired glucose tolerance (IGT), impaired fasting blood glucose (IFG), insulin resistance and/or from metabolic syndrome to type 2 diabetes mellitus;
- preventing, reducing the risk of, slowing the progression of, delaying the onset of or treating of complications of diabetes mellitus such as micro- and macrovascular diseases, such as nephropathy, micro- or macroalbuminuria, proteinuria, retinopathy, cataracts, neuropathy, learning or memory impairment, neurodegenerative or cognitive disorders, cardio- or cerebrovascular diseases, tissue ischaemia, diabetic foot or ulcus, atherosclerosis, hypertension, endothelial dysfunction, myocardial infarction, acute coronary syndrome, unstable angina pectoris, stable angina pectoris, peripheral arterial occlusive disease, cardiomyopathy, heart failure, heart rhythm disorders, vascular restenosis, and/or stroke;
- reducing body weight and/or body fat or preventing an increase in body weight and/or body fat or facilitating a reduction in body weight and/or body fat;
- preventing, slowing the progression of, delaying the onset of or treating the degeneration of pancreatic beta cells and/or the decline of the functionality of pancreatic beta cells and/or for improving, preserving and/or restoring the functionality of pancreatic beta cells and/or stimulating and/or restoring or protecting the functionality of pancreatic insulin secretion and/or increasing pancreatic beta cell mass;
- preventing, slowing the progression of, delaying the onset of or treating non alcoholic fatty liver disease (NAFLD) including hepatic steatosis, non-alcoholic steatohepatitis (NASH) and/or liver fibrosis (such as e.g. preventing, slowing the progression, delaying, attenuating, treating or reversing hepatic steatosis, (hepatic) inflammation and/or an abnormal accumulation of liver fat);
- preventing, slowing the progression of, delaying the onset of or treating type 2 diabetes with primary or secondary failure to conventional (oral or non-oral) antihyperglycemic mono- or combination therapy or delaying the need for insulin treatment;
- achieving a reduction in the dose of conventional antihyperglycemic medication required for adequate therapeutic effect;
- reducing the risk for adverse effects associated with conventional antihyperglycemic medication; and/or
- maintaining and/or improving the insulin sensitivity and/or for treating or preventing hyperinsulinemia and/or insulin resistance;
particularly either in a drug-naïve type 2 diabetes patient or in a type 2 diabetes patient with insufficient glycemic control despite therapy with one or two conventional antihyperglycemic agents selected from metformin, sulphonylureas, thiazolidinediones, glinides, alpha-glucosidase blockers, GLP-1 or GLP-1 analogues, and insulin or insulin analogues; optionally in combination with one or more other active substances, such as e.g. any of those mentioned herein.

In a particular embodiment, the present disclosure provides a method of treating and/or preventing metabolic diseases, particularly type 2 diabetes mellitus and conditions related thereto (e.g. diabetic complications), in drug-naïve patients (e.g. as first line therapy); said method comprising administering to a subject in need thereof (particularly a human patient) an effective amount of a pharmaceutically composition of BI 1356 and metformin according to this disclosure.

In another particular embodiment, the present disclosure provides a method of treating and/or preventing metabolic diseases, particularly type 2 diabetes mellitus and conditions related thereto (e.g. diabetic complications), in patients with insufficient glycemic control despite mono-therapy with metformin (e.g. as second line therapy); said method comprising administering to a subject in need thereof (particularly a human patient) an effective amount of a pharmaceutically composition of BI 1356 and metformin according to this disclosure.

In another particular embodiment, the present disclosure provides a method of treating and/or preventing metabolic diseases, particularly type 2 diabetes mellitus and conditions related thereto (e.g. diabetic complications), in patients with insufficient glycemic control despite dual combination therapy with metformin and a thiazolidinedione (e.g. as third line therapy); said method comprising administering to a subject in need thereof (particularly a human patient) an effective amount of a pharmaceutically composition of BI 1356 and metformin according to this disclosure, and a thiazolidinedione.

In another particular embodiment, the present disclosure provides a method of treating and/or preventing metabolic diseases, particularly type 2 diabetes mellitus and conditions related thereto (e.g. diabetic complications), in patients with insufficient glycemic control despite dual combination therapy with metformin and a sulphonylurea (e.g. as third line therapy); said method comprising administering to a subject in need thereof (particularly a human patient) an effective amount of a pharmaceutically composition of BI 1356 and metformin according to this disclosure, and a sulphonylurea.

In a further embodiment, the present disclosure provides a method of treating and/or preventing metabolic diseases, particularly type 2 diabetes mellitus and conditions related thereto (e.g. diabetic complications), in patients with insufficient glycemic control despite dual combination therapy with metformin and insulin or insulin analog; said method comprising administering to a subject in need thereof (particularly a human patient) an effective amount of a pharmaceutically composition of BI 1356 and metformin according to this disclosure, and insulin or insulin analog.

In a further embodiment, the present disclosure provides a method of treating and/or preventing metabolic diseases, particularly type 2 diabetes mellitus and conditions related thereto (e.g. diabetic complications), in patients treated with insulin or insulin analog; said method comprising administering to a subject in need thereof (particularly a human patient) an effective amount of a pharmaceutically composition of BI 1356 and metformin according to this disclosure, thereby replacing said insulin or insulin analog (i.e. switching from insulin therapy to a BI 1356 & metformin combination according to this disclosure).

Examples of such metabolic diseases or disorders amenable to the therapy of this disclosure may include, without being restricted to, Type 1 diabetes, Type 2 diabetes, inadequate glucose tolerance, insulin resistance, hyperglycemia, hyperlipidemia, hypercholesterolemia, dyslipidemia, metabolic syndrome X, obesity, hypertension, chronic systemic inflammation, non-alcoholic fatty liver disease (NAFLD), retinopathy, neuropathy, nephropathy, atherosclerosis, endothelial dysfunction and osteoporosis.

In the monitoring of the treatment of diabetes mellitus the HbA1c value, the product of a non-enzymatic glycation of the haemoglobin B chain, is of exceptional importance. As its formation depends essentially on the blood sugar level and the life time of the erythrocytes the HbA1c in the sense of a "blood sugar memory" reflects the average blood sugar level of the preceding 4-12 weeks. Diabetic patients whose HbA1c level has been well controlled over a long time by more intensive diabetes treatment (i.e. < 6.5 % of the total haemoglobin in the sample) are significantly better protected from diabetic microangiopathy. The available treatments for diabetes can give the diabetic an average improvement in their HbA1c level of the order of 1.0 - 1.5 %. This reduction in the HbA1C level is not sufficient in all diabetics to bring them into the desired target range of < 7.0 %, preferably < 6.5 % and more preferably < 6 % HbA1c.

Within glycemic control, in addition to improvement of the HbA1c level, other recommended therapeutic goals for type 2 diabetes mellitus patients are improvement of fasting plasma glucose (FPG) and of postprandial plasma glucose (PPG) levels to normal or as near normal as possible. Recommended desired target ranges of preprandial (fasting) plasma glucose are 90-130 mg/dL (or 70-130 mg/dL) or <110 mg/dL, and of two-hour postprandial plasma glucose are <180 mg/dL or <140 mg/dL.

Within the meaning of this disclosure, inadequate or insufficient glycemic control means in particular a condition wherein patients show HbA1c values above 6.5%, in particular above 7.0%, even more preferably above 7.5%, especially above 8%. An embodiment of patients with inadequate or insufficient glycemic control include, without being limited to, patients having a HbA1c value from 7.5 to 10% (or, in another embodiment, from 7.5 to 11%). Another embodiment of patients with inadequate or insufficient glycemic control include, without being limited to, patients having HbA1c value from 6.5 to 8.4% (stage 1), or, in yet another embodiment, from 8.5 to 9.4% (stage 2), or, in still yet another embodiment, ≥ 9.5% (stage 3). A special sub-embodiment of inadequately controlled patients refers to patients with poor glycemic control including, without being limited, patients having a HbA1c value ≥ 9%.

In one embodiment, diabetes patients within the meaning of this disclosure may include patients who have not previously been treated with an antidiabetic drug (drug-naive patients). Thus, in an embodiment, the therapies described herein may be used in naïve patients. In another embodiment, diabetes patients within the meaning of this disclosure may include patients with advanced or late stage type 2 diabetes mellitus (including patients with failure to conventional antidiabetic therapy), such as e.g. patients with inadequate glycemic control on one, two or more conventional oral and/or non-oral antidiabetic drugs as defined herein, such as e.g. patients with insufficient glycemic control despite (mono-)therapy with metformin, a thiazolidinedione (particularly pioglitazone), a sulphonylurea, a glinide, GLP-1 or GLP-1 analogue, insulin or insulin analogue, or an α-glucosidase inhibitor, or despite dual combination therapy with metformin/sulphonylurea, metformin/thiazolidinedione (particularly pioglitazone), sulphonylurea/ α-glucosidase inhibitor, pioglitazone/sulphonylurea, metformin/insulin, pioglitazone/insulin or sulphonylurea/insulin. Thus, in an embodiment, the therapies described herein may be used in patients experienced with therapy, e.g. with conventional oral and/or non-oral antidiabetic mono- or dual or triple combination medication as mentioned herein.

An embodiment of the patients which may be amenable to the therapies of this disclosure may include, without being limited, those diabetes patients for whom normal metformin therapy is not appropriate, such as e.g. those diabetes patients who need reduced dose metformin therapy due to reduced tolerability, intolerability or contraindication against metformin or due to (mildly) impaired/reduced renal function (including elderly patients, such as e.g. ≥ 60-65 years).

A special embodiment of the DPP-4 inhibitors of this disclosure refers to those orally administered DPP-4 inhibitors which are therapeutically efficacious at low dose levels, e.g. at dose levels < 100 mg or < 70 mg per patient per day, preferably < 50 mg, more preferably < 30 mg or < 20 mg, even more preferably from 1 mg to 10 mg (if required, divided into 1 to 4 single doses, particularly 1 or 2 single doses, which may be of the same size), particularly from 1 mg to 5 mg (more particularly 5 mg), per patient per day, preferentially, administered orally once-daily, more preferentially, at any time of day, administered with or without food. Thus, for example, the daily oral amount 5 mg BI 1356 can be given in a once daily dosing regimen (i.e. 5 mg BI 1356 once daily) or in a twice daily dosing regimen (i.e. 2.5 mg BI 1356 twice daily), at any time of day, with or without food.

A particularly preferred DPP-4 inhibitor to be emphasized within the meaning of this disclosure is 1-[(4-methyl-quinazolin-2-yl)methyl]-3-methyl-7-(2-butyn-1-yl)-8-(3-(R)-amino-piperidin-1-yl)-xanthine (also known as BI 1356 or linagliptin). BI 1356 exhibits high potency, 24h duration of action, and a wide therapeutic window. In patients with type 2 diabetes receiving multiple oral doses of 1, 2.5, 5 or 10 mg of BI 1356 once daily for 12 days, BI 1356 shows favourable pharmacodynamic and pharmacokinetic profile with rapid attainment of steady state (e.g. reaching steady state plasma levels (> 90% of the pre-dose plasma concentration on Day 13) between second and fifth day of treatment in all dose groups), little accumulation (e.g. with a mean accumulation ratio R_{A,AUC} ≤ 1.4 with doses above 1 mg) and preserving a long-lasting effect on DPP-4 inhibition (e.g. with almost complete (> 90%) DPP-4 inhibition at the 5 mg and 10 mg dose levels, i.e. 92.3 and 97.3% inhibition at steady state, respectively, and > 80% inhibition over a 24h interval after drug intake), as well as significant decrease in 2h postprandial blood glucose excursions by ≥ 80 % (already on Day 1) in doses ≥ 2.5 mg, and with the cumulative amount of unchanged parent compound excreted in urine on Day 1 being below 1% of the administered dose and increasing to not more than about 3-6% on Day 12 (renal clearance CL_{R,ss} is from about 14 to about 70 mL/min for the administered oral doses, e.g. for the 5 mg dose renal clearance is about 70 ml/min). In people with type 2 diabetes BI 1356 shows a placebo-like safety and tolerability (e.g. low risk for hypoglycemia, edema or weight gain). With low doses of about ≥ 5 mg, BI 1356 acts as a true once-daily oral drug with a full 24 h duration of DPP-4 inhibition. At therapeutic oral dose levels, BI 1356 is mainly excreted via the liver and only to a minor extent (about < 7% of the administered oral dose) via the kidney. BI 1356 is primarily excreted unchanged via the bile. The fraction of BI 1356 eliminated via the kidneys increases only very slightly over time and with increasing dose, so that there will likely be no need to modify the dose of BI 1356 based on the patients' renal function. The non-renal elimination of BI 1356 in combination with its low accumulation potential and broad safety margin may be of significant benefit in a patient population that has a high prevalence of renal insufficiency and diabetic nephropathy. BI 1356 is suitable for once-daily dosing without the need for dose titration when co-administered with metformin.

In one embodiment, pharmaceutical compositions or fixed dose combinations of this disclosure include, without being limited to, such compositions which comprise immediate release metformin and linagliptin (preferably linagliptin as an immediate release component). Examples of such compositions include, without being limited, mono-layer tablets, bi-layer tablets, tablets-in-tablets/Bull's eye tablets or drug (linagliptin)-coated tablets (each of which may be optionally over-coated with a non-functional film-coat), e.g. such tablet forms as described in more detail herein, particularlyl those given in the example section (preferred is hereby the mono-layer tablet of this disclosure).

In another embodiment, pharmaceutical compositions or fixed dose combinations of this disclosure include, without being limited to, such compositions which comprise controlled or sustained (e.g. slow or extended) release metformin and linagliptin (preferably linagliptin as an immediate release component). Examples of such compositions include, without being limited, drug (linagliptin)-coated tablets (which may be optionally over-coated with a non-functional film-coat), e.g. compositions comprising i) an extended release core comprising metformin and one or more suitable excipients and ii) a (preferably immediate release) film-coating comprising linagliptin (e.g. such a film-coat layer as described herein). Examples of slow release include, without being limited, a metformin composition (e.g. as tablet core) where metformin is released at a rate where the peak plasma levels of metformin are typically achieved about 8-22 h after administration.

Typical dosage strengths of the dual fixed dose combination (tablet) of linagliptin / metformin IR (immediate release) are 2.5/500 mg, 2.5/850 mg and 2.5/1000 mg, which may be administered 1-3 times a day, particularly twice a day.

Typical dosage strengths of the dual fixed dose combination (tablet) of linagliptin / metformin XR (extended release) are 5/500 mg, 5/1000 mg and 5/1500 mg, which may be administered 1-2 times a day, particularly once a day (preferably to be taken in the evening preferably with meal, e.g. prior to sleep), or 2.5/500, 2.5/750 and 2.5/1000, which may be administered 1-2 times a day, particularly one or two tablets once a day (preferably to be taken in the evening preferably with meal).

Metformin is usually given in doses varying from about 500 mg to 2000 mg up to 2500 mg per day using various dosing regimens from about 100 mg to 500 mg or 200 mg to 850 mg (1-3 times a day), or about 300 mg to 1000 mg once or twice a day, or delayed-release metformin in doses of about 100 mg to 1000 mg or preferably 500 mg to 1000 mg once or twice a day or about 500 mg to 2000 mg once a day. Particular dosage strengths may be 250, 500, 625, 750, 850 and 1000 mg of metformin hydrochloride.

As different metabolic functional disorders often occur simultaneously, it is quite often indicated to combine a number of different active principles with one another. Thus, depending on the functional disorders diagnosed, improved treatment outcomes may be obtained if a DPP-4 inhibitor or pharmaceutical composition according to this disclosure is combined with active substances customary for the respective disorders, such as e.g. one or more active substances selected from among the other antidiabetic substances, especially active substances that lower the blood sugar level or the lipid level in the blood, raise the HDL level in the blood, lower blood pressure or are indicated in the treatment of atherosclerosis or obesity.

The DPP-4 inhibitors or pharmaceutical compositions mentioned herein - besides their use on their own - may also be used in conjunction with other active substances, by means of which improved treatment results can be obtained. Such a combined treatment may be given as a free combination of the substances or in the form of a fixed combination, for example in a tablet or capsule. Pharmaceutical formulations of the combination partner needed for this may either be obtained commercially as pharmaceutical compositions or may be formulated by the skilled man using conventional methods. The active substances which may be obtained commercially as pharmaceutical compositions are described in numerous places in the prior art, for example in the list of drugs that appears annually, the "Rote Liste ^{®}" of the federal association of the pharmaceutical industry, or in the annually updated compilation of manufacturers' information on prescription drugs known as the "Physicians' Desk Reference".

Examples of antidiabetic combination partners (beyond metformin) are sulphonylureas such as glibenclamide, tolbutamide, glimepiride, glipizide, gliquidon, glibornuride and gliclazide; nateglinide; repaglinide; thiazolidinediones such as rosiglitazone and pioglitazone; PPAR gamma modulators such as metaglidases; PPAR-gamma agonists such as rivoglitazone, mitoglitazone, INT-131 or balaglitazone; PPAR-gamma antagonists; PPAR-gamma/alpha modulators such as tesaglitazar, muraglitazar, aleglitazar, indeglitazar and KRP297; PPAR-gamma/alpha/delta modulators such as e.g. lobeglitazone; AMPK-activators such as AICAR; acetyl-CoA carboxylase (ACC1 and ACC2) inhibitors; diacylglycerol-acetyltransferase (DGAT) inhibitors; pancreatic beta cell GCRP agonists such as SMT3-receptor-agonists and GPR119, such as the GPR119 agonists 5-ethyl-2-{4-[4-(4-tetrazol-1-yl-phenoxymethyl)-thiazol-2-yl]-piperidin-1-yl}-pyrimidine or 5-[1-(3-isopropyl-[1,2,4]oxadiazol-5-yl)-piperidin-4-ylmethoxy]-2-(4-methanesulfonyl-phenyl)-pyridine; 11ß-HSD-inhibitors; FGF19 agonists or analogues; alpha-glucosidase blockers such as acarbose, voglibose and miglitol; alpha2-antagonists; insulin and insulin analogues such as human insulin, insulin lispro, insulin glusilin, r-DNA-insulinaspart, NPH insulin, insulin detemir, insulin degludec, insulin tregopil, insulin zinc suspension and insulin glargin; Gastric inhibitory Peptide (GIP); amylin and amylin analogues (e.g. pramlintide, davalintide); or GLP-1 and GLP-1 analogues such as Exendin-4, e.g. exenatide, exenatide LAR, liraglutide, taspoglutide, lixisenatide (AVE-0010), LY-2428757, dulaglutide (LY-2189265), semaglutide or albiglutide; SGLT2-inhibitors such as dapagliflozin, sergliflozin (KGT-1251), atigliflozin, canagliflozin, ipragliflozin or tofogliflozin; inhibitors of protein tyrosine-phosphatase (e.g. trodusquemine); inhibitors of glucose-6-phosphatase; fructose-1,6-bisphosphatase modulators; glycogen phosphorylase modulators; glucagon receptor antagonists; phosphoenolpyruvatecarboxykinase (PEPCK) inhibitors; pyruvate dehydrogenasekinase (PDK) inhibitors; inhibitors of tyrosine-kinases (50 mg to 600 mg) such as PDGF-receptor-kinase (cf. EP-A-564409, WO 98/35958, US 5093330, WO 2004/005281, and WO 2006/041976) or of serine/threonine kinase; glucokinase/regulatory protein modulators incl. glucokinase activators; glycogen synthase kinase inhibitors; inhibitors of the SH2-domain-containing inositol 5-phosphatase type 2 (SHIP2) ; IKK inhibitors such as high-dose salicylate ; JNK1 inhibitors ; protein kinase C-theta inhibitors; beta 3 agonists such as ritobegron, YM 178, solabegron, talibegron, N-5984, GRC-1087, rafabegron, FMP825; aldosereductase inhibitors such as AS 3201, zenarestat, fidarestat, epalrestat, ranirestat, NZ-314, CP-744809, and CT-112; SGLT-1 or SGLT-2 inhibitors, such as e.g. dapagliflozin, sergliflozin, atigliflozin or canagliflozin (or compound of formula (I-S) or (I-K) from WO 2009/035969); KV 1.3 channel inhibitors; GPR40 modulators such as e.g. [(3S)-6-({2',6'-dimethyl-4'-[3-(methylsulfonyl)propoxy]biphenyl-3-yl}methoxy)-2,3-dihydro-1-benzofuran-3-yl]acetic acid; SCD-1 inhibitors; CCR-2 antagonists; dopamine receptor agonists (bromocriptine mesylate [Cycloset]); 4-(3-(2,6-dimethylbenzyloxy)phenyl)-4-oxobutanoic acid; sirtuin stimulants; and other DPP IV inhibitors.

A dosage of pioglitazone is usually of about 1-10 mg, 15 mg, 30 mg, or 45 mg once a day.

Rosiglitazone is usually given in doses from 4 to 8 mg once (or divided twice) a day (typical dosage strengths are 2, 4 and 8 mg).

Glibenclamide (glyburide) is usually given in doses from 2.5-5 to 20 mg once (or divided twice) a day (typical dosage strengths are 1.25, 2.5 and 5 mg), or micronized glibenclamide in doses from 0.75-3 to 12 mg once (or divided twice) a day (typical dosage strengths are 1.5, 3, 4.5 and 6 mg).

Glipizide is usually given in doses from 2.5 to 10-20 mg once (up to 40 mg divided twice) a day (typical dosage strengths are 5 and 10 mg), or extended-release glipizide in doses from 5 to 10 mg (up to 20 mg) once a day (typical dosage strengths are 2.5, 5 and 10 mg).

Glimepiride is usually given in doses from 1-2 to 4 mg (up to 8 mg) once a day (typical dosage strengths are 1, 2 and 4 mg).

A dual combination of glibenclamide/metformin is usually given in doses from 1.25/250 once daily to 10/1000 mg twice daily (typical dosage strengths are 1.25/250, 2.5/500 and 5/500 mg).

A dual combination of glipizide/metformin is usually given in doses from 2.5/250 to 10/1000 mg twice daily (typical dosage strengths are 2.5/250, 2.5/500 and 5/500 mg).

A dual combination of glimepiride/metformin is usually given in doses from 1/250 to 4/1000 mg twice daily.

A dual combination of rosiglitazone/glimepiride is usually given in doses from 4/1 once or twice daily to 4/2 mg twice daily (typical dosage strengths are 4/1, 4/2, 4/4, 8/2 and 8/4 mg). A dual combination of pioglitazone/glimepiride is usually given in doses from 30/2 to 30/4 mg once daily (typical dosage strengths are 30/4 and 45/4 mg).

A dual combination of rosiglitazone/metformin is usually given in doses from 1/500 to 4/1000 mg twice daily (typical dosage strengths are 1/500, 2/500, 4/500, 2/1000 and 4/1000 mg).

A dual combination of pioglitazone/metformin is usually given in doses from 15/500 once or twice daily to 15/850 mg thrice daily (typical dosage strengths are 15/500 and 15/850 mg).

The non-sulphonylurea insulin secretagogue nateglinide is usually given in doses from 60 to 120 mg with meals (up to 360 mg/day, typical dosage strengths are 60 and 120 mg); repaglinide is usually given in doses from 0.5 to 4 mg with meals (up to 16 mg/day, typical dosage strengths are 0.5, 1 and 2 mg). A dual combination of repaglinide/metformin is available in dosage strengths of 1/500 and 2/850 mg.

Acarbose is usually given in doses from 25 to 100 mg with meals (up to 300 mg/day, typical dosage strengths are 25, 50 and 100 mg). Miglitol is usually given in doses from 25 to 100 mg with meals (up to 300 mg/day, typical dosage strengths are 25, 50 and 100 mg).

Conventional antidiabetics and antihyperglycemics typically used in mono- or dual or triple (add-on or initial) combination therapy may include, without being limited to, metformin, sulphonylureas, thiazolidinediones, glinides, alpha-glucosidase blockers, GLP-1 and GLP-1 analogues, as well as insulin and insulin analogues, such as e.g. those agents indicated herein by way of example, including combinations thereof.

Examples of combination partners that lower the lipid level in the blood are HMG-CoA-reductase inhibitors such as simvastatin, atorvastatin, lovastatin, fluvastatin, pravastatin, pitavastatin and rosuvastatin; fibrates such as bezafibrate, fenofibrate, clofibrate, gemfibrozil, etofibrate and etofyllinclofibrate; nicotinic acid and the derivatives thereof such as acipimox; PPAR-alpha agonists; PPAR-delta agonists; inhibitors of acyl-coenzyme A:cholesterolacyltransferase (ACAT; EC 2.3.1.26) such as avasimibe; cholesterol resorption inhibitors such as ezetimib; substances that bind to bile acid, such as cholestyramine, colestipol and colesevelam; inhibitors of bile acid transport; HDL modulating active substances such as D4F, reverse D4F, LXR modulating active substances and FXR modulating active substances; CETP inhibitors such as torcetrapib, JTT-705 (dalcetrapib) or compound 12 from WO 2007/005572 (anacetrapib); LDL receptor modulators; and ApoB100 antisense RNA.

A dosage of atorvastatin is usually from 1 mg to 40 mg or 10 mg to 80 mg once a day.

Examples of combination partners that lower blood pressure are beta-blockers such as atenolol, bisoprolol, celiprolol, metoprolol and carvedilol; diuretics such as hydrochlorothiazide, chlortalidon, xipamide, furosemide, piretanide, torasemide, spironolactone, eplerenone, amiloride and triamterene; calcium channel blockers such as amlodipine, nifedipine, nitrendipine, nisoldipine, nicardipine, felodipine, lacidipine, lercanipidine, manidipine, isradipine, nilvadipine, verapamil, gallopamil and diltiazem; ACE inhibitors such as ramipril, lisinopril, cilazapril, quinapril, captopril, enalapril, benazepril, perindopril, fosinopril and trandolapril; as well as angiotensin II receptor blockers (ARBs) such as telmisartan, candesartan, valsartan, losartan, irbesartan, olmesartan, azilsartan and eprosartan.

A dosage of telmisartan is usually from 20 mg to 320 mg or 40 mg to 160 mg per day.

Examples of combination partners which increase the HDL level in the blood are Cholesteryl Ester Transfer Protein (CETP) inhibitors; inhibitors of endothelial lipase; regulators of ABC1; LXRalpha antagonists; LXRbeta agonists; PPAR-delta agonists; LXRalpha/beta regulators, and substances that increase the expression and/or plasma concentration of apolipoprotein A-I.

Examples of combination partners for the treatment of obesity are sibutramine; tetrahydrolipstatin (orlistat); alizyme; dexfenfluramine; axokine; cannabinoid receptor 1 antagonists such as the CB1 antagonist rimonobant; MCH-1 receptor antagonists; MC4 receptor agonists; NPY5 as well as NPY2 antagonists; beta3-AR agonists such as SB-418790 and AD-9677; 5HT2c receptor agonists such as APD 356 (lorcaserin); myostatin inhibitors; Acrp30 and adiponectin; steroyl CoA desaturase (SCD1) inhibitors; fatty acid synthase (FAS) inhibitors; CCK receptor agonists; Ghrelin receptor modulators; Pyy 3-36; orexin receptor antagonists; and tesofensine; as well as the dual combinations bupropion/naltrexone, bupropion/zonisamide, topiramate/phentermine and pramlintide/metreleptin.

Examples of combination partners for the treatment of atherosclerosis are phospholipase A2 inhibitors; inhibitors of tyrosine-kinases (50 mg to 600 mg) such as PDGF-receptor-kinase (cf. EP-A-564409, WO 98/35958, US 5093330, WO 2004/005281, and WO 2006/041976); oxLDL antibodies and oxLDL vaccines; apoA-1 Milano; ASA; and VCAM-1 inhibitors.

### Pharmaceutical compositions, formulations, tablets comprising such formulations and process for their preparation according to this disclosure:

The present disclosure refers to pharmaceutical compositions comprising fixed dose combinations of a DPP-4 inhibitor drug and the partner drug metformin, and processes for the preparation thereof.

In a more detailed aspect, the present disclosure refers to oral solid dosage forms for fixed dose combination (FDC) of a selected dipeptidyl peptidase-4 (DPP-4) inhibitor drug and the partner drug metformin. The FDC formulations are chemically stable and either a) display similarity of in-vitro dissolution profiles and/or are bioequivalent to the free combination, or b) allow to adjust the in-vitro and in-vivo performance to desired levels. In a preferred embodiment the disclosure refers to chemically stable FDC formulations maintaining the original dissolution profiles of corresponding mono tablets of each individual entity, with a reasonable tablet size.

The enzyme DPP-4 also known as CD26 is a serine protease known to lead to the cleavage of a dipeptide from the N-terminal end of a number of proteins having at their N-terminal end a prolin or alanin residue. Due to this property DPP-4 inhibitors interfere with the plasma level of bioactive peptides including the peptide GLP-1 and are considered to be promising drugs for the treatment of diabetes mellitus.

For example, DPP-4 inhibitors and their uses are disclosed in WO 2002/068420, WO 2004/018467, WO 2004/018468, WO 2004/018469, WO 2004/041820, WO 2004/046148, WO 2005/051950, WO 2005/082906, WO 2005/063750, WO 2005/085246, WO 2006/027204, WO 2006/029769 or WO2007/014886; or in WO 2004/050658, WO 2004/111051, WO 2005/058901, WO 2005/097798; WO 2006/068163, WO 2007/071738, WO 2008/017670; WO 2007/128721, WO 2007/128724 or WO 2007/128761, or WO 2009/121945.

The biguanide antihyperglycemic agent metformin is disclosed in US patent No. 3,174,901.The preparation of metformin (dimethyldiguanide) and its hydrochloride salt is state of the art and was disclosed first by Emil A. Werner and James Bell, J. Chem. Soc. 121, 1922, 1790-1794. Other pharmaceutically acceptable salts of metformin can be found in US application Serial No. 09/262,526 filed March 4, 1999 or US patent No. 3,174,901. It is preferred that the metformin employed herein be the metformin hydrochloride salt.

Unless specifically noted, in the present context the terms "DPP-4 inhibitor(s)", "biguanide(s)", or any species thereof like "metformin", are also intended to comprise any pharmaceutically acceptable salt thereof, crystal form, hydrate, solvate, diastereomer or enantiomer thereof.

In attempts to prepare pharmaceutical compositions of selected DPP-4 inhibitors it has been observed, that the DPP-4 inhibitors with a primary or secondary amino group show incompatibilities, degradation problems, or extraction problems with a number of customary excipients such as microcrystalline cellulose, sodium starch glycolate, croscarmellose sodium, tartaric acid, citric acid, glucose, fructose, saccharose, lactose, maltodextrines. Though the compounds themselves are very stable, they react with incompatible partner drug, or its impurity product, and/or with many excipients used in solid dosage forms and with impurities of excipients, especially in tight contact provided in tablets and at high excipient/drug ratios. The amino group appears to react with reducing sugars and with other reactive carbonyl groups and with carboxylic acid functional groups formed for example at the surface of microcrystalline cellulose by oxidation. These unforeseen difficulties are primarily observed in low dosage ranges of the DPP-4 inhibitor used, which are required due to their surprising potency, and/or high dosage ranges of the partner drug used. Thus, pharmaceutical compositions are required to solve these technical problems, which may be associated with the unexpected potency of selected DPP-4 inhibitor compounds.

Other aims of the present disclosure will become apparent to the skilled man from the foregoing and following remarks.

It has now been found that the pharmaceutical compositions, which are described in greater details herein, have surprising and particularly advantageous properties.

In particular, it has been found that by the use of a nucleophilic and/or basic agent, which may be suitable for stabilizing, such as e.g. a suitable buffering agent as stabilizer, within these pharmaceutical compositions one can overcome these problems, e.g. of incompatibility and poor stability, especially decomposition and/or "assay decrease" which may be caused e.g. by reaction (e.g. by acylation, urea formation or Maillard reaction, or the like) of free base type DPP-4 inhibitors when combined with an incompatible partner drug, or its impurity product and/or a pharmaceutical excipient having such functional group (such as a reducing end of a sugar or an acyl group, such as e.g. an acetyl or carbamoyl group) to form derivatives with the free base type DPP-4 inhibitors, such as e.g. N-acetyl or N-carbamoyl derivatives. Therefore, by the use of a suitable nucleophilic and/or basic agent (e.g. a buffering and/or pH modifying agent) within these pharmaceutical compositions protection against decomposition and degradation can be achieved.

Thus, the present disclosure is directed to a chemically stable FDC formulation comprising a DPP-4 inhibitor, a partner drug, and a nucleophilic and/or basic agent.

Thus, the present disclosure is also directed to a chemically stable FDC formulation comprising a DPP-4 inhibitor, a partner drug, and a suitable buffering agent.

Thus, the present disclosure is also directed to a chemically stable FDC formulation comprising a DPP-4 inhibitor, a partner drug, and a pH modifying agent.

A DPP-4 inhibitor within the meaning of the present disclosure includes, without being limited to, any of those DPP-4 inhibitors mentioned hereinabove and hereinbelow, preferably orally active DPP-4 inhibitors.

In a closer embodiment, a DPP-4 inhibitor within the meaning of the present disclosure includes a DPP-4 inhibitor with an amino group, especially a free or primary amino group.

In a yet closer embodiment, a DPP-4 inhibitor in the context of the present disclosure is a DPP-4 inhibitor with a primary amino group, particularly with a free primary amino group.

The partner drug used is metformin, particularly metformin hydrochloride (1,1-dimethylbiguanide hydrochloride or metformin HCI).

The buffering agent used may be a basic amino acid, which has an intramolecular amino group and alkaline characteristics (isoelectric point, pI: 7.59-10.76), such as e.g. L-arginine, L-lysine or L-histigine. A preferred buffering agent within the meaning of this disclosure is L-arginine. L-Arginine has a particular suitable stabilizing effect on the compositions of this disclosure, e.g. by suppressing degradation of the DPP-4 inhibitor in the presence of the partner drug.

The present disclosure is directed to a pharmaceutical comprising a DPP-4 inhibitor, a partner drug, a nucleophilic and/or basic agent, and one or more pharmaceutical excipients.

The present disclosure is also directed to a pharmaceutical composition comprising a DPP-4 inhibitor, a partner drug, a suitable buffering agent, and one or more pharmaceutical excipients.

The present disclosure is also directed to a pharmaceutical comprising a DPP-4 inhibitor, a partner drug, a pH modifying agent, and one or more pharmaceutical excipients.

In an embodiment, the present disclosure is directed to a pharmaceutical composition (e.g. an oral solid dosage form, particularly a tablet) comprising a DPP-4 inhibitor; a partner drug (particularly metformin); and L-arginine for stabilizing the composition and/or the DPP-4 inhibitor, particularly against chemical degradation; as well as one or more pharmaceutical excipients.

In another embodiment, the present disclosure is directed to a pharmaceutical composition (e.g. an oral solid dosage form, particularly a tablet) obtainable from a DPP-4 inhibitor; a partner drug (particularly metformin); and L-arginine for stabilizing the composition and/or the DPP-4 inhibitor, particularly against chemical degradation; as well as one or more pharmaceutical excipients.

In general, pharmaceutical excipients which may be used may be selected from the group consisting of one or more fillers, one or more binders or diluents, one or more lubricants, one or more disintegrants, and one or more glidants, one or more film-coating agents, one or more plasticizers, one or more pigments, and the like.

The pharmaceutical compositions (tablets) of this disclosure comprise usually a binder.

In more detail, the pharmaceutical compositions (tablets) of this disclosure comprise usually one or more fillers (e.g. D-mannitol, corn starch and/or pregelatinized starch), a binder (e.g. copovidone), a lubricant (e.g. magnesium stearate), and a glidant (e.g. colloidal anhydrous silica).

Suitably the pharmaceutical excipients used within this disclosure are conventional materials such as D-mannitol, corn starch, pregelatinized starch as a filler, copovidone as a binder, magnesium stearate as a lubricant, colloidal anhydrous silica as a glidant, hypromellose as a film-coating agent, propylene glycol as a plasticizer, titanium dioxide, iron oxide red/yellow as a pigment, and talc, etc.

A typical composition according to the present disclosure comprises the binder copovidone (also known as copolyvidone or Kollidon VA64).

Further, a typical composition according to the present disclosure comprises the filler corn starch, the binder copovidone, the lubricant magnesium stearate, and the glidant colloidal anhydrous silica.

A pharmaceutical composition according to an embodiment of the present disclosure is intended for the treatment of diabetes and/or to achieve glycemic control in a type 1 or type 2 diabetes mellitus patient and comprises a fixed dose combination formulation as described herein together with suitable pharmaceutical excipients. Additionally the compositions can be used to treat rheumatoid arthritis, obesity and osteoporosis as well as to support allograft transplantation.

Thus, in particular, the present disclosure is directed to a pharmaceutical composition (especially an oral solid dosage form, particularly a tablet) comprising a DPP-4 inhibitor, metformin hydrochloride, L-arginine and one or more pharmaceutical excipients, particularly one or more fillers, one or more binders, one or more glidants, and/or one or more lubricants.

In more particular, the present disclosure is directed to a pharmaceutical composition (especially an oral solid dosage form, particularly a tablet) comprising a DPP-4 inhibitor, metformin hydrochloride, L-arginine, copovidone as binder and one or more further pharmaceutical excipients.

Typical pharmaceutical compositions of this disclosure may comprise in the DPP-4 inhibitor portion 0.1-10 % L-arginine (such as e.g. about 0.1 %, 0.25 %, 0.556 %, 2.12 %, 2.22 % or 10 %) by weight of total DPP-4 inhibitor portion, particularly about 2 % (e.g. more specifically, 2.12 % by weight of total tablet core of uncoated monolayer tablet).

Typical pharmaceutical compositions of this disclosure may comprise in the DPP-4 inhibitor portion (% by weight of total DPP-4 inhibitor portion):
0.2-10 % DPP-4 inhibitor, and
0.1-10% L-arginine.

Typical pharmaceutical compositions of this disclosure may comprise the DPP-4 inhibitor and L-arginine in a weight ratio of from about 1:20 to about 10:1 or from about 1:15 to about 10:1 or from about 1:10 to about 10:1, especially from 1:10 to 5:2, such as e.g. in a weight ratio of 1:10, 1:8.5, 1:5, 1:1, or 1:0.4, more detailed in a weight ratio of 2.5mg:25mg, 2.5mg:21.2mg, 2.5mg:12.5mg, 2.5mg:2.5mg, or 2.5mg:1mg.

Typical pharmaceutical compositions of this disclosure may comprise metformin hydrochloride and L-arginine in a weight ratio of from about 40:1 to about 1000:1, such as e.g. in a weight ratio of 40:1, 200:1, 340:1, 400:1, 500:1, 850:1, or 1000:1, more detailed in a weight ratio of 500mg:12.5mg, 850mg:21.2mg, 1000mg:25mg, 500mg:2.5mg, 850mg:2.5mg, 1000mg:2.5mg, 500mg:1mg, 850mg:1mg, or 1000mg:1mg.

Typical pharmaceutical compositions of this disclosure may comprise the DPP4-inhibitor, metformin hydrochloride and L-arginine in a weight ratio of from about 1:200:0.4 to about 1:200:5 (e.g. 1:200:0.4, 1:200:1, 1:200:5), or from about 1:340:0.4 to about 1:340:8.5 (e.g. 1:340:0.4, 1:340:1, 1:340:8.5), or from about 1:400:0.4 to about 1:400:10 (e.g. 1:400:0.4, 1:400:1, 1:400:10).

Typical pharmaceutical compositions of this disclosure may comprise one or more of the following amounts (% by weight of total coated tablet mass):

| | |
|---|---|
| 0.1-0.5 % | DPP-4 inhibitor, |
| 47-85 % | metformin HCI, |
| 0.07-2.2 % | L-arginine, |
| 3.9-8.1 % | binder (e.g. copovidone), |
| 2.3-5.9 % | filler 1 (e.g. corn starch), |
| 0-4.4 % | filler 2 (e.g. pregelatinized starch), |
| 0-33 % | filler 3 (e.g. D-mannitol), |
| 0.7-1.5 % | lubricant (e.g. magnesium stearate), and |
| 0.1-0.5 % | glidant (e.g. colloidal anhydrous silica). |

Further details about the FDC formulations of this disclosure, e.g. the ingredients, ratio of ingredients (such as e.g. ratio of DPP-4 inhibitor, metformin hydrochloride, L-arginine and/or excipients), particularly with respect to special dosage forms (tablets) used within this disclosure as well as their preparation, become apparent to the skilled person from the disclosure hereinbefore and hereinafter (including by way of example the following examples as well as the claims).

In a first embodiment (embodiment A), a DPP-4 inhibitor in the context of the present disclosure is any DPP-4 inhibitor of
formula (I) 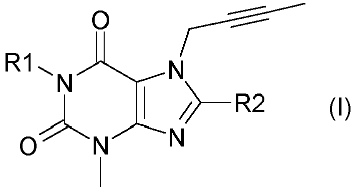
or formula (II) 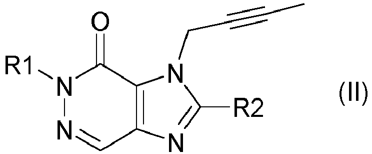
or formula (III) 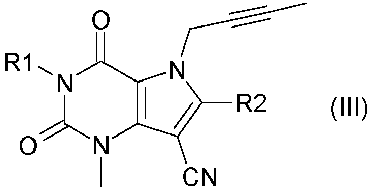
wherein **R1** denotes ([1,5]naphthyridin-2-yl)methyl, (quinazolin-2-yl)methyl, (quinoxalin-6-yl)methyl, (4-methyl-quinazolin-2-yl)methyl, 2-cyano-benzyl, (3-cyano-quinolin-2-yl)methyl, (3-cyano-pyridin-2-yl)methyl, (4-methyl-pyrimidin-2-yl)methyl, or (4,6-dimethyl-pyrimidin-2-yl)methyl and **R2** denotes 3-(*R*)-amino-piperidin-1-yl, (2-amino-2-methyl-propyl)-methylamino or (2-(*S*)-amino-propyl)-methylamino,
or its pharmaceutically acceptable salt;

In a second embodiment (embodiment **B**), a DPP-4 inhibitor in the context of the present disclosure is a DPP-4 inhibitor selected from the group consisting of
sitagliptin, vildagliptin, saxagliptin and alogliptin,
or its pharmaceutically acceptable salt.

Regarding the first embodiment (embodiment **A**), preferred DPP-4 inhibitors are any or all of the following compounds and their pharmaceutically acceptable salts:
- 1-[(4-methyl-quinazolin-2-yl)methyl]-3-methyl-7-(2-butyn-1-yl)-8-(3-(*R*)-amino-piperidin-1-yl)-xanthine (compare WO 2004/018468, example 2(142): 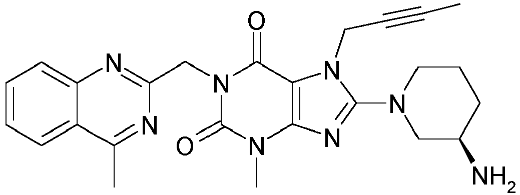
- 1-[([1,5]naphthyridin-2-yl)methyl]-3-methyl-7-(2-butyn-1-yl)-8-((*R*)-3-amino-piperidin-1-yl)-xanthine (compare WO 2004/018468, example 2(252)): 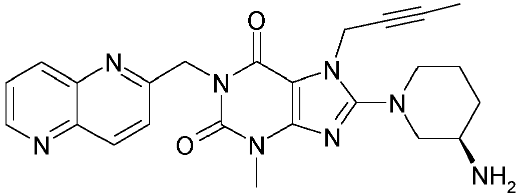
- 1-[(Quinazolin-2-yl)methyl]-3-methyl-7-(2-butyn-1-yl)-8-((*R*)-3-amino-piperidin-1-yl)-xanthine (compare WO 2004/018468, example 2(80)): 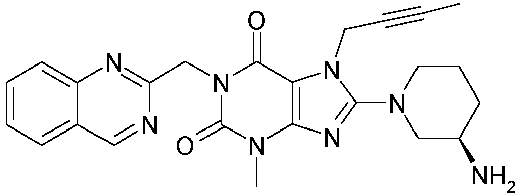
- 2-((R)-3-Amino-piperidin-1-yl)-3-(but-2-yinyl)-5-(4-methyl-quinazolin-2-ylmethyl)-3,5-dihydro-imidazo[4,5-d]pyridazin-4-one (compare WO 2004/050658, example 136): 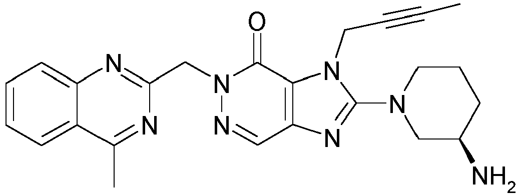
- 1-[(4-Methyl-quinazolin-2-yl)methyl]-3-methyl-7-(2-butyin-1-yl)-8-[(2-amino-2-methylpropyl)-methylamino]-xanthine (compare WO 2006/029769, example 2(1)): 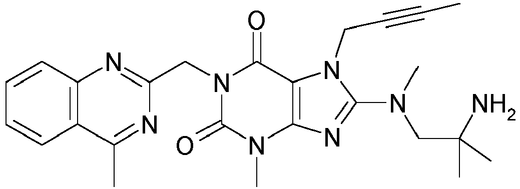
- 1-[(3-Cyano-quinolin-2-yl)methyl]-3-methyl-7-(2-butyn-1-yl)-8-((*R*)-3-amino-piperidin-1-yl)-xanthine (compare WO 2005/085246, example 1(30)): 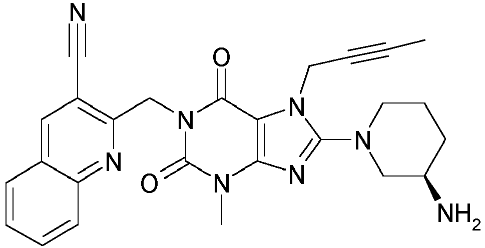
- 1-(2-Cyano-benzyl)-3-methyl-7-(2-butyn-1-yl)-8-((*R*)-3-amino-piperidin-1-yl)-xanthine (compare WO 2005/085246, example 1(39)): 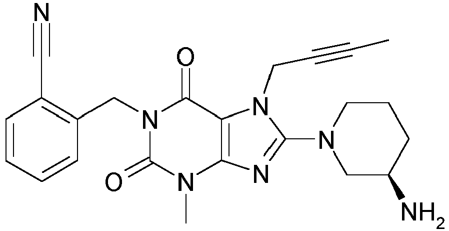
- 1-[(4-Methyl-quinazolin-2-yl)methyl]-3-methyl-7-(2-butyn-1-yl)-8-[(*S*)-(2-amino-propyl)-methylamino]-xanthine (compare WO 2006/029769, example 2(4)): 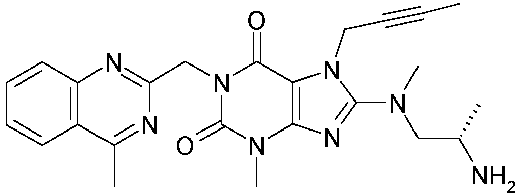
- 1-[(3-Cyano-pyridin-2-yl)methyl]-3-methyl-7-(2-butyn-1-yl)-8-((*R*)-3-amino-piperidin-1-yl)-xanthine (compare WO 2005/085246, example 1(52)): 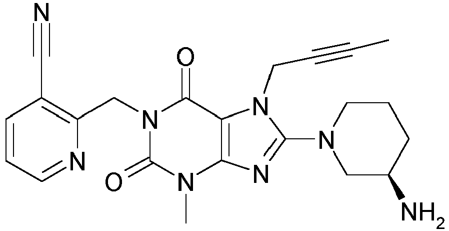
- 1-[(4-Methyl-pyrimidin-2-yl)methyl]-3-methyl-7-(2-butyn-1-yl)-8-((*R*)-3-amino-piperidin-1-yl)-xanthine (compare WO 2005/085246, example 1(81)): 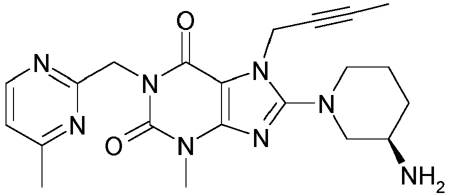
- 1-[(4,6-Dimethyl-pyrimidin-2-yl)methyl]-3-methyl-7-(2-butyn-1-yl)-8-((*R*)-3-amino-piperidin-1-yl)-xanthine (compare WO 2005/085246, example 1(82)): 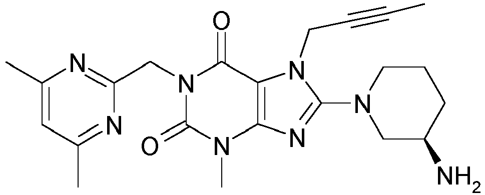
- 1-[(Quinoxalin-6-yl)methyl]-3-methyl-7-(2-butyn-1-yl)-8-((*R*)-3-amino-piperidin-1-yl)-xanthine (compare WO 2005/085246, example 1(83)):

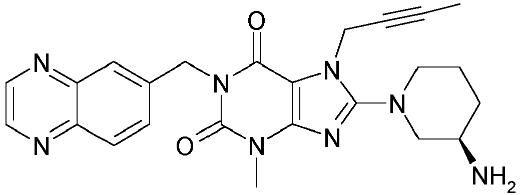

These DPP-4 inhibitors are distinguished from structurally comparable DPP-4 inhibitors, as they combine exceptional potency and a long-lasting effect with favourable pharmacological properties, receptor selectivity and a favourable side-effect profile or bring about unexpected therapeutic advantages or improvements when combined with other pharmaceutical active substances. Their preparation is disclosed in the publications mentioned.

A more preferred DPP-4 inhibitor among the abovementioned DPP-4 inhibitors of embodiment **A** of this disclosure is 1-[(4-methyl-quinazolin-2-yl)methyl]-3-methyl-7-(2-butyn-1-yl)-8-(3-(*R*)-amino-piperidin-1-yl)-xanthine, particularly the free base thereof (which is also known as BI 1356).

Regarding the second embodiment (embodiment **B**), preferred DPP-4 inhibitors are selected from the group consisting of vildagliptin, saxagliptin and alogliptin, and their pharmaceutically acceptable salts.

Unless otherwise noted, according to this disclosure it is to be understood that the definitions of the above listed DPP-4 inhibitors also comprise their pharmaceutically acceptable salts as well as hydrates, solvates and polymorphic forms thereof. With respect to salts, hydrates and polymorphic forms thereof, particular reference is made to those which are referred to hereinabove and hereinbelow.

With respect to embodiment **A,** the methods of synthesis for the DPP-4 inhibitors according to embodiment **A** of this disclosure are known to the skilled person. Advantageously, the DPP-4 inhibitors according to embodiment **A** of this disclosure can be prepared using synthetic methods as described in the literature. Thus, for example, purine derivatives of formula (I) can be obtained as described in WO 2002/068420, WO 2004/018468, WO 2005/085246, WO 2006/029769 or WO 2006/048427.

Purine derivatives of formula (II) can be obtained as described, for example, in WO 2004/050658 or WO 2005/110999.

Purine derivatives of formula (III) can be obtained as described, for example, in WO 2006/068163, WO 2007/071738 or WO 2008/017670. The preparation of those DPP-4 inhibitors, which are specifically mentioned hereinabove, is disclosed in the publications mentioned in connection therewith. Polymorphous crystal modifications and formulations of particular DPP-4 inhibitors are disclosed in WO 2007/128721 and WO 2007/128724, respectively.

With respect to embodiment **B**, the methods of synthesis for the DPP-4 inhibitors of embodiment **B** are described in the scientific literature and/ or in published patent documents, particularly in those cited herein.

With respect to the first embodiment (embodiment **A**), the dosage typically required of the DPP-4 inhibitors mentioned herein in embodiment **A** when administered orally is 0.5 mg to 100 mg, preferably 2.5 mg to 50 mg or 0.5 mg to 10 mg, more preferably 2.5 mg to 10 mg or 1 mg to 5 mg, in each case 1 to 4 times a day. Thus, the dosage required of 1-[(4-methyl-quinazolin-2-yl)methyl]-3-methyl-7-(2-butyn-1-yl)-8-(3-(R)-amino-piperidin-1-yl)-xanthine when administered orally is 0.5 mg to 10 mg per patient per day, preferably 2.5 mg to 10 mg or 1 mg to 5 mg per patient per day.

A dosage form prepared with a pharmaceutical composition comprising a DPP-4 inhibitor mentioned herein in embodiment **A** contain the active ingredient in a dosage range of 0.1-100 mg, in particular 0.5 to 10 mg. Thus, particular dosage strengths of 1-[(4-methyl-quinazolin-2-yl)methyl]-3-methyl-7-(2-butyn-1-yl)-8-(3-(R)-amino-piperidin-1-yl)-xanthine are 0.5 mg, 1 mg, 2.5 mg, 5 mg and 10 mg. A more particular unit dosage strength of 1-[(4-methyl-quinazolin-2-yl)methyl]-3-methyl-7-(2-butyn-1-yl)-8-(3-(R)-amino-piperidin-1-yl)-xanthine for inclusion into fixed dose combination pharmaceutical compositions of the present disclosure is 2.5 mg.

With respect to the second embodiment (embodiment **B**), the doses of DPP-4 inhibitors mentioned herein in embodiment **B** to be administered to mammals, for example human beings, of, for example, approximately 70 kg body weight, may be generally from about 0.5 mg to about 350 mg, for example from about 10 mg to about 250 mg, preferably 20-200 mg, more preferably 20-100 mg, of the active moiety per person per day, or from about 0.5 mg to about 20 mg, preferably 2.5-10 mg, per person per day, divided preferably into 1 to 4 single doses which may, for example, be of the same size. Single dosage strengths comprise, for example, 2.5, 5, 10, 25, 40, 50, 75, 100, 150 and 200 mg of the DPP-4 inhibitor active moiety.

A dosage strength of the DPP-4 inhibitor sitagliptin is usually between 25 and 200 mg of the active moiety. A recommended dose of sitagliptin is 100 mg calculated for the active moiety (free base anhydrate) once daily. Unit dosage strengths of sitagliptin free base anhydrate (active moiety) are 25, 50, 75, 100, 150 and 200 mg. Particular unit dosage strengths of sitagliptin (e.g. per tablet) are 25, 50 and 100 mg. An equivalent amount of sitagliptin phosphate monohydrate to the sitagliptin free base anhydrate is used in the pharmaceutical compositions, namely, 32.13, 64.25, 96.38, 128.5, 192.75, and 257 mg, respectively. Adjusted dosages of 25 and 50 mg sitagliptin are used for patients with renal failure.

A dosage range of the DPP-4 inhibitor vildagliptin is usually between 10 and 150 mg daily, in particular between 25 and 150 mg, 25 and 100 mg or 25 and 50 mg or 50 and 100 mg daily. Particular examples of daily oral dosage are 25, 30, 35, 45, 50, 55, 60, 80, 100 or 150 mg. In a more particular aspect, the daily administration of vildagliptin is between 25 and 150 mg or between 50 and 100 mg. In another more particular aspect, the daily administration of vildagliptin is 50 or 100 mg. The application of the active ingredient may occur up to three times a day, preferably one or two times a day. Particular dosage strengths are 50 mg or 100 mg vildagliptin.

Metformin is usually given in doses varying from about 250 mg to 3000 mg, particularly from 500 mg to 2000 mg up to 2500 mg per day using various dosage regimens.

A dosage range of the partner drug metformin is usually from 100 mg to 500 mg or 200 mg to 850 mg (1-3 times a day), or from 300 mg to 1000 mg once or twice a day.

The unit dosage strengths of the metformin hydrochloride for use in the present disclosure may be from 100 mg to 2000 mg or from 250 mg to 2000 mg, preferably from 250 mg to 1000 mg. Particular dosage strengths may be 250, 500, 625, 750, 850 and 1000 mg of metformin hydrochloride. These unit dosage strengths of metformin hydrochloride represent the dosage strengths approved in the US for marketing to treat type 2 diabetes. More particular unit dosage strengths of metformin hydrochloride for incorporation into the fixed dose combination pharmaceutical compositions of the present disclosure are 500, 850 and 1000 mg of metformin hydrochloride.

The amount of the DPP-4 inhibitor and of the partner drug in the pharmaceutical composition according to this disclosure correspond to the respective dosage ranges as provided hereinbefore. For example, a pharmaceutical composition comprises 1-[(4-methyl-quinazolin-2-yl)methyl]-3-methyl-7-(2-butyn-1-yl)-8-(3-(R)-amino-piperidin-1-yl)-xanthine in an amount of 0.5 mg to 10 mg (namely 0.5 mg, 1 mg, 2.5 mg, 5 mg or 10 mg) and of metformin hydrochloride in an amount of 250 mg to 1000 mg (namely 250, 500, 625, 750, 850 or 1000 mg).

Specific embodiments of dosage strengths for 1-[(4-methyl-quinazolin-2-yl)methyl]-3-methyl-7-(2-butyn-1-yl)-8-(3-(R)-amino-piperidin-1-yl)-xanthine and metformin hydrochloride in the fixed dose combinations of the present disclosure are the following:
(1) 2.5 mg of 1-[(4-methyl-quinazolin-2-yl)methyl]-3-methyl-7-(2-butyn-1-yl)-8-(3-(R)-amino-piperidin-1-yl)-xanthine free base, and 500 mg metformin hydrochloride;
(2) 2.5 mg of 1-[(4-methyl-quinazolin-2-yl)methyl]-3-methyl-7-(2-butyn-1-yl)-8-(3-(R)-amino-piperidin-1-yl)-xanthine free base, and 850 mg metformin hydrochloride;
(3) 2.5 mg of 1-[(4-methyl-quinazolin-2-yl)methyl]-3-methyl-7-(2-butyn-1-yl)-8-(3-(R)-amino-piperidin-1-yl)-xanthine free base, and 1000 mg metformin hydrochloride.

The particular fixed dose combinations of BI 1356 and metformin of the present disclosure may be administered once or twice daily to the patient, in particular twice daily.

In a preferred aspect of the present disclosure, the present disclosure is directed to a pharmaceutical composition (especially an oral solid dosage form, particularly a tablet) comprising or obtainable from
a DPP-4 inhibitor selected from the group consisting of 1-[(4-methyl-quinazolin-2-yl)methyl]-3-methyl-7-(2-butyn-1-yl)-8-(3-(R)-amino-piperidin-1-yl)-xanthine free base, vildagliptin, saxagliptin and alogliptin,
metformin hydrochloride,
L-arginine,
and one or more pharmaceutical excipients, such as e.g. those described herein.

A particularly preferred DPP-4 inhibitor to be emphasized within the meaning of this disclosure is 1-[(4-methyl-quinazolin-2-yl)methyl]-3-methyl-7-(2-butyn-1-yl)-8-(3-(R)-amino-piperidin-1-yl)-xanthine free base (also known as BI 1356).

In particular, it has been found that L-arginine is effective as stabilizing agent for FDC combinations of 1-[(4-methyl-quinazolin-2-yl)methyl]-3-methyl-7-(2-butyn-1-yl)-8-(3-(R)-amino-piperidin-1-yl)-xanthine free base with metformin HCI. Even after 6 months storage at accelerated conditions L-arginine is able to suppress degradation of 1-[(4-methyl-quinazolin-2-yl)methyl]-3-methyl-7-(2-butyn-1-yl)-8-(3-(R)-amino-piperidin-1-yl)-xanthine free base effectively. The effect seems to be concentration dependent. Thus, L-arginine may act as stabilizing and buffering agent in the formulation.

In a more preferred aspect of the present disclosure, the present disclosure is directed to a pharmaceutical composition (especially an oral solid dosage form, particularly a tablet) comprising or made from
1-[(4-methyl-quinazolin-2-yl)methyl]-3-methyl-7-(2-butyn-1-yl)-8-(3-(R)-amino-piperidin-1-yl)-xanthine free base (BI 1356),
metformin hydrochloride,
L-arginine,
and one or more pharmaceutical excipients, such as e.g. those described herein.

Typical pharmaceutical compositions according to this disclosure comprise or are made by comprising combining any one of the following amounts (1), (2) or (3) of active ingredients and L-arginine:
(1) 2.5 mg of 1-[(4-methyl-quinazolin-2-yl)methyl]-3-methyl-7-(2-butyn-1-yl)-8-(3-(R)-amino-piperidin-1-yl)-xanthine free base, 500 mg metformin hydrochloride, and from 1.0 mg to 12.5 mg L-arginine (specifically 1.0 mg, 2.5 mg or 12.5 mg L-arginine);
(2) 2.5 mg of 1-[(4-methyl-quinazolin-2-yl)methyl]-3-methyl-7-(2-butyn-1-yl)-8-(3-(R)-amino-piperidin-1-yl)-xanthine free base, 850 mg metformin hydrochloride, and from 1.0 mg to 21.2 mg L-arginine (specifically 1.0 mg, 2.5 mg or 21.2 mg L-arginine);
(3) 2.5 mg of 1-[(4-methyl-quinazolin-2-yl)methyl]-3-methyl-7-(2-butyn-1-yl)-8-(3-(R)-amino-piperidin-1-yl)-xanthine free base, 1000 mg metformin hydrochloride, and from 1.0 mg to 25.0 mg L-arginine (specifically 1.0 mg, 2.5 mg or 25 mg L-arginine).

In a further aspect of the present disclosure, the present disclosure provides methods of manufacturing of the compositions, formulations, blends or dosage forms of this disclosure, such as e.g. by using methods known to one skilled in the art and/or in a manner as described herein, for example they may be obtained by processes comprising using (e.g. mixing, combining, blending and/or composing) the components and/or ingredients, or pre-mixtures thereof, mentioned hereinbefore and hereinafter, as well as the present disclosure further provides compositions, formulations, blends or dosage forms obtainable by these methods or processes and/or obtainable from the components, ingredients, pre-mixtures and/or mixtures mentioned hereinbefore and hereinafter.

In a further aspect of the present disclosure, the present disclosure provides a pharmaceutical composition, formulation, blend or dosage form of this disclosure which is substantially free of or only marginally comprises impurities and/or degradation products; that means, for example, that the composition, formulation, blend or dosage from includes about <5%, or about <4%, or about <3%, or less than about 2%, preferably less than about 1%, more preferably less than about 0.5%, even more preferably less than about 0.2% of any individual or total impurity or degradation product(s) by total weight, such as e.g. N-acetyl, N-formyl, N-methyl and/or N-carbamoyl derivative of the free base type DPP-4 inhibitor. The content and/or degradation can be determined by well-known analytical methods, for example using HPLC methods.

In this context, in a further aspect of the present disclosure, the present disclosure provides derivatives of a DPP-4 inhibitor having an amino group, particularly a free primary amino group, as mentioned herein, said derivatives being obtainable by acetylation of the amino group (e.g. to yield the group -NHC(O)CH₃) or by carbamoylation of the amino group (e.g. to yield the group -NHC(O)NH₂) or by formylation of the amino group (e.g. to yield the group - NHC(O)H) or by methylation of the amino group (e.g. to yield the group -NHCH₃). Compositions, formulations and dosage forms such as described herein comprising one or more of such derivatives (e.g. in trace amounts and mixed with the respective DPP-4 inhibitors indicated herein) or substantially free thereof are also contemplated.

### Dosage forms for the FDC formulations of this disclosure:

Another purpose of this disclosure is to develop the FDC formulations of this disclosure with a reasonable tablet size, with good tablet properties (e.g. stability, hardness, friability, disintegration, content uniformity and the like) and, in a preferred embodiment, without disturbing the original dissolution profiles of each mono tablet in case of desired proof of bioequivalence with minimized risk of failure.

Designing of the dosage form is an important matter not only to optimize the tablet size and dissolution profiles but also to minimize the amount of stabilizing agent, because the pH change by dissolving of buffering agent may affect the dissolution profiles of the DPP-4 inhibitor or a partner drug. The selection of the dosage form is depending on the dose strengths of the active ingredients used and their physicochemical and solid state characteristics.

A conventional approach (i.e. physical separation) may not be useful for stabilization of certain DPP-4 inhibitors of this disclosure. A buffering agent like L-arginine need to be added into the formulation for suppressing degradation, however it may be necessary to minimize the amount of L-arginine because its alkaline characteristics give a negative impact on the dissolution profiles or the stability of the DPP-4 inhibitor or a partner drug.

Thus, it has been found that suitable dosage forms for the FDC formulations of this disclosure are film-coated tablets (film-coating for drug loading, such as particularly DPP-4 inhibitor drug loading by film coating on tablet cores containing the partner drug), mono-layer tablets, bi-layer tablets, tri-layer tablets and press-coated tablets (e.g. tablet-in-tablet or bull's eye tablet with DPP-4 inhibitor core) , which dosage forms are good measures to achieve the goal under consideration of desired pharmaceutical profiles and characteristics of a DPP-4 inhibitor and a partner drug used.

Said dosage forms have been found to be applicable to the FDC formulations either keeping the original dissolution profiles of each mono tablet or adjusting the profiles to desired levels, e.g. including extended release characteristics, and a reasonable tablet size.

A typical mono-layer tablet of this disclosure comprises a DPP-4 inhibitor, metformin hydrochloride, L-arginine, one or more fillers (such as e.g. corn starch), one or more binders (such as e.g. copovidone), one or more glidants (such as e.g. colloidal anhydrous silica) and one or more lubricants (such as e.g. magnesium stearate).

In a preferred embodiment of the present disclosure, the present disclosure is directed to an oral solid pharmaceutical composition, preferably a tablet, particularly a mono-layer tablet comprising or made from
1-[(4-methyl-quinazolin-2-yl)methyl]-3-methyl-7-(2-butyn-1-yl)-8-(3-(R)-amino-piperidin-1-yl)-xanthine (also known as BI 1356, e.g. in an amount of 2.5 mg),
metformin (particularly metformin hydrochloride, e.g. in an amount of 500 mg, 850 mg or 1000 mg),
L-arginine,
and one or more pharmaceutical excipients, particularly one or more fillers (e.g. corn starch), one or more binders (e.g. copovidone), one or more glidants (e.g. colloidal anhydrous silica) and/or one or more lubricants (e.g. magnesium stearate),
as well as, optionally, a film coat e.g. comprising one or more film-coating agents (e.g. hypromellose), one or more plasticizers (e.g. propylene glycol), one or more pigments (e.g. titanium dioxide, iron oxide red and/or iron oxide yellow) and/or one or more glidants (e.g. talc).

A method of manufacturing a tablet of this disclosure comprises tabletting (e.g. compression) of one or more final blends in form of granules. Granules of the (final) blend(s) according to this disclosure may be prepared by methods well-known to one skilled in the art (e.g. high shear wet granulation or fluid bed granulation). Granules according to this disclosure as well as details of granulation processes (including their separate steps) for the preparation of granules of this disclosure are described by way of example in the following examples.

An illustrative granulation process for the preparation of granules comprising the mono-layer composition comprises
i.) combining (e.g. dissolving or dispersing) L-arginine, a binder (e.g. copovidone) and, optionally, the DPP-4 inhibitor (e.g. BI 1356) in a solvent or mixture of solvents such as purified water at ambient temperature to produce a granulation liquid;
ii.) blending metformin HCI, a filler (e.g. corn starch) and, optionally, the DPP-4 inhibitor (e.g. BI 1356) in a suitable mixer (e.g. fluid-bed granulator) to produce a pre-mix;
   wherein the DPP-4 inhibitor (e.g. BI 1356) may be included either in the granulation liquid obtained in i.) or in the pre-mix obtained in ii.), preferably BI 1356 is dispersed in the granulation liquid and is absent in the pre-mix;
iii.) spraying the granulation-liquid into the pre-mix and granulating the mixture for example in a fluid-bed granulator, preferably under dry condition;
iv.) drying the granulate, e.g. at about 70° C inlet air temperature until the desired loss on drying value in the range of 1-2 % is obtained;
v.) delumping the dried granulate for example by sieving through a sieve with a mesh size of 0.5 to 1.0 mm;
vi.) blending the sieved granulate and preferably sieved glidant (e.g. colloidal anhydrous silica) in a suitable blender;
vii.) adding preferably sieved lubricant (e.g. magnesium stearate) to the granulate for final blending for example in the free-fall blender.

Preferentially, a mono-layer tablet according to this disclosure comprises or is obtainable from a mixture comprising any one of the following amounts (1), (2) or (3) of active ingredients and L-arginine:
(1) 2.5 mg of 1-[(4-methyl-quinazolin-2-yl)methyl]-3-methyl-7-(2-butyn-1-yl)-8-(3-(R)-amino-piperidin-1-yl)-xanthine free base, 500 mg metformin hydrochloride, and 12.5 mg L-arginine;
(2) 2.5 mg of 1-[(4-methyl-quinazolin-2-yl)methyl]-3-methyl-7-(2-butyn-1-yl)-8-(3-(R)-amino-piperidin-1-yl)-xanthine free base, 850 mg metformin hydrochloride, and 21.2 mg L-arginine;
(3) 2.5 mg of 1-[(4-methyl-quinazolin-2-yl)methyl]-3-methyl-7-(2-butyn-1-yl)-8-(3-(R)-amino-piperidin-1-yl)-xanthine free base, 1000 mg metformin hydrochloride, and 25 mg L-arginine.

A typical bi-layer tablet of this disclosure comprises
a DPP-4 inhibitor portion comprising a DPP-4 inhibitor, L-arginine, one or more fillers (such as e.g. D-mannitol, pregelatinized starch and corn starch), one or more binders (such as e.g. copovidone) and one or more lubricants (such as e.g. magnesium stearate),
and
a metformin HCI portion comprising metformin hydrochloride, one or more fillers (such as e.g. corn starch), one or more binders (such as e.g. copovidone), one or more glidants (such as e.g. colloidal anhydrous silica) and one or more lubricants (such as e.g. magnesium stearate).

Preferentially, a bi-layer tablet according to this disclosure comprises or is obtainable from a mixture comprising any one of the following amounts (1), (2) or (3) of active ingredients and L-arginine:
(1) 2.5 mg of 1-[(4-methyl-quinazolin-2-yl)methyl]-3-methyl-7-(2-butyn-1-yl)-8-(3-(R)-amino-piperidin-1-yl)-xanthine free base, 500 mg metformin hydrochloride, and 2.5 mg L-arginine;
(2) 2.5 mg of 1-[(4-methyl-quinazolin-2-yl)methyl]-3-methyl-7-(2-butyn-1-yl)-8-(3-(R)-amino-piperidin-1-yl)-xanthine free base, 850 mg metformin hydrochloride, and 2.5 mg L-arginine;
(3) 2.5 mg of 1-[(4-methyl-quinazolin-2-yl)methyl]-3-methyl-7-(2-butyn-1-yl)-8-(3-(R)-amino-piperidin-1-yl)-xanthine free base, 1000 mg metformin hydrochloride, and 2.5 mg L-arginine.

A typical press-coated tablet (tablet-in-tablet or bull's eye tablet) of this disclosure comprises a DPP-4 inhibitor core portion comprising a DPP-4 inhibitor, L-arginine, one or more fillers (such as e.g. D-mannitol, pregelatinized starch and corn starch), one or more binders (such as e.g. copovidone) and one or more lubricants (such as e.g. magnesium stearate), and
a metformin HCI portion comprising metformin hydrochloride, one or more fillers (such as e.g. corn starch), one or more binders (such as e.g. copovidone), one or more glidants (such as e.g. colloidal anhydrous silica) and one or more lubricants (such as e.g. magnesium stearate).

Preferentially, a press-coated tablet (tablet-in-tablet or bull's eye tablet) according to this disclosure comprises or is obtainable from a mixture comprising any one of the following amounts (1), (2) or (3) of active ingredients and L-arginine:
(1) 2.5 mg of 1-[(4-methyl-quinazolin-2-yl)methyl]-3-methyl-7-(2-butyn-1-yl)-8-(3-(R)-amino-piperidin-1-yl)-xanthine free base, 500 mg metformin hydrochloride, and 1.0 mg L-arginine;
(2) 2.5 mg of 1-[(4-methyl-quinazolin-2-yl)methyl]-3-methyl-7-(2-butyn-1-yl)-8-(3-(R)-amino-piperidin-1-yl)-xanthine free base, 850 mg metformin hydrochloride, and 1.0 mg L-arginine;
(3) 2.5 mg of 1-[(4-methyl-quinazolin-2-yl)methyl]-3-methyl-7-(2-butyn-1-yl)-8-(3-(R)-amino-piperidin-1-yl)-xanthine free base, 1000 mg metformin hydrochloride, and 1.0 mg L-arginine.

A typical film-coated tablet (DPP-4 inhibitor coating on metformin HCI tablet, i.e. drug layering by film-coating for drug loading) of this disclosure comprises
a metformin HCI core portion comprising metformin hydrochloride, one or more fillers (such as e.g. corn starch), one or more binders (such as e.g. copovidone), one or more glidants (such as e.g. colloidal anhydrous silica) and one or more lubricants (such as e.g. magnesium stearate),
wherein said core portion is seal-coated with a film coat comprising one or more film-coating agents (such as e.g. hypromellose), one or more plasticizers (such as e.g. propylene glycol), one or more pigments (such as e.g. titanium dioxide, iron oxide red and/or iron oxide yellow) and one or more glidants (such as e.g. talc);
and
a DPP-4 inhibitor layer comprising a DPP-4 inhibitor, L-arginine, one or more film-coating agents (such as e.g. hypromellose) and one or more plasticizers (such as e.g. propylene glycol).

Preferentially, a film-coated tablet (DPP4-inhibitor drug loading) according to this disclosure comprises or is obtainable from a mixture comprising any one of the following amounts (1), (2) or (3) of active ingredients and L-arginine:
(1) 2.5 mg of 1-[(4-methyl-quinazolin-2-yl)methyl]-3-methyl-7-(2-butyn-1-yl)-8-(3-(R)-amino-piperidin-1-yl)-xanthine free base, 500 mg metformin hydrochloride, and 2.5 mg L-arginine;
(2) 2.5 mg of 1-[(4-methyl-quinazolin-2-yl)methyl]-3-methyl-7-(2-butyn-1-yl)-8-(3-(R)-amino-piperidin-1-yl)-xanthine free base, 850 mg metformin hydrochloride, and 2.5 mg L-arginine;
(3) 2.5 mg of 1-[(4-methyl-quinazolin-2-yl)methyl]-3-methyl-7-(2-butyn-1-yl)-8-(3-(R)-amino-piperidin-1-yl)-xanthine free base, 1000 mg metformin hydrochloride, and 2.5 mg L-arginine.

Preferably, these abovementioned tablets (mono-, bi-layer, press-coated and drug-coated tablets) are further over-coated with a final film coat, which comprises a film-coating agent (such as e.g. hypromellose), a plasticizer (such as e.g. propylene glycol), pigments (such as e.g. titanium dioxide, iron oxide red and/or iron oxide yellow) and a glidant (such as e.g. talc). Typically this additional film over-coat may represent 1-4 %, preferentially 1-2 %, of the total mass of the composition.

The following dosage forms of the disclosure can be applied to the FDC formulation of 1-[(4-methyl-quinazolin-2-yl)methyl]-3-methyl-7-(2-butyn-1-yl)-8-(3-(R)-amino-piperidin-1-yl)-xanthine free base (BI 1356) and metformin hydrochloride based on the characteristics of drug substances and requirements of the desired pharmaceutical profiles:

### a) Mono-layer tablets

Mono-layer tablets with L-arginine show satisfactory stability results, good dissolution properties and good content uniformity (CU). Mono-layer tablets can be manufactured using conventional technologies (including fluid-bed granulation for the DPP-4 inhibitor and metformin hydrochloride, e.g. comprising adding the DPP-4 inhibitor as powder or as an aqueous suspension in the granulation liquid to the fluid bed granulator).

### b) Bi-layer tablets

Bi-layer tablets with L-arginine show promising stability results, good dissolution properties and good CU. Bi-layer tablets can be manufactured using conventional bi-layer tableting technologies (e.g. rotary bi-layer tableting machine).

### c) Press-coated tablets

Press-coated tablets (tablet-in-tablets and advanced press-coated bull's eye tablets) show promising stability, good CU and dissolution. Press-coated tablets can be manufactured using conventional press-coating technology, such as e.g. on a Kilian tablet press to obtain tablet-in-tablet or on other conventional press-coater to obtain bull's eye tablet. As an advantage of this approach, it is easy to minimize the amount of L-arginine in the formulation and control the assay and CU of the DPP-4 inhibitor portion (very small amount of drug loading; 2.5 mg/tablet where the dose strengths of metformin HCI are 500, 850 and 1000 mg/tablet). Another advantage is that DPP-4 inhibitor- and metformin HCI- portion can be designed flexibly to minimize the tablet size. A modified press-coated tablet named "bull's eye tablet" may be a universal dosage potentially for bi-layer tablets as well as other FDC. Bull's eye tablet can be manufactured in a one-step press-coating without separate core formation (like in bi-layer tableting) being necessary.

It is to be noted that within the meaning of this disclosure the skilled person is aware about what is meant with the phrase "bull's eye tablet" used herein. As it known to the skilled person, this tablet (also referred to as an inlay tablet or a dot) is composed of an outer coat and an inner core, and in which, instead of the inner core zone being completely surrounded by the outer coat, one surface of the zone corresponding to the inner core zone is exposed.

### d) Film-coated tablets (drug layering by film-coating for drug loading)

Coating of DPP-4 inhibitor drug substance on the metformin HCI tablets shows acceptable dissolution results and promising stability data. L-arginine needs to be added into film-coating for stabilization. As an advantage for this approach, it is possible to integrate DPP-4 inhibitor portion into a partner drug portion as it is, even if the dosage form is a modified/controlled release formulation. Within the film-coating process coating endpoint determination is necessary via analytics.

The method of layering of the DPP-4 inhibitor by film-coating as described herein (including the steps of seal-coating, drug-loading and, optional, over-coating) may be applied to any kind of cores or tablets which may comprise an active ingredient (e.g. a partner drug as mentioned herein), for example metformin cores or tablets, such as e.g. immediate release metformin tablets, sustained release metformin tablets, extended release metformin tablets, modified release metformin tablets, controlled release metformin tablets or delayed release metformin tablets. Thus, the present disclosure further relates to a tablet which comprises a film-coat layer comprising the DPP-4 inhibitor, a film-forming agent (e.g. hypromellose), a plasticizer (e.g. propylene glycol) and L-arginine, or which is obtainable by comprising using such a method of layering of the DPP-4 inhibitor by film-coating as described herein. The present disclosure also relates to a FDC tablet comprising an immediate or extended release metformin tablet core, a seal coat, a film-coat layer comprising the DPP-4 inhibitor, and, optionally, an over-coat; e.g. each as described herein, as well as to such a FDC tablet made by a process comprising the following steps of seal-coating on a metformin tablet core, layering of a DPP-4 inhibitor by film-coating and, optional, over-coating, e.g. each step such as described herein.

Pharmaceutical immediate release dosage forms of this disclosure preferably have dissolution properties such that after 45 minutes for each of the active ingredients at least 75 %, even more preferably at least 90 % by weight of the respective active ingredient is dissolved. In a particular embodiment, after 30 minutes for each of the active ingredients especially of the mono-layer tablet according to this disclosure (including tablet core and film-coated tablet) at least 70-75 % (preferably at least 80 %) by weight of the respective active ingredient is dissolved. In a further embodiment, after 15 minutes for each of the active ingredients especially of the mono-layer tablet according to this disclosure (including tablet core and film-coated tablet) at least 55-60 % by weight of the respective active ingredient is dissolved. The dissolution properties can be determined in standard dissolution tests, e.g. according to standard pharmacopeias (e.g. using paddle method with agitation speed of 50 rpm, 0.1M hydrochloric acid as dissolution medium at a temperature of 37°C, and HPLC (BI 1356) and UV (metformin) analysis of the samples).

In the pharmaceutical compositions and pharmaceutical dosage forms according to the disclosure BI 1356, for example a crystalline form thereof, preferably has a particle size distribution (preferably by volume) such that at least 90 % of the respective active pharmaceutical ingredient has a particle size smaller than 200 µm, i.e. X90 < 200 µm, more preferably X90 ≤ 150 µm. More preferably the particle size distribution is such that X90 ≤ 100 µm, even more preferably X90 ≤ 75 µm. In addition the particle size distribution is preferably such that X90 > 0.1 µm, more preferably X90 ≥ 1 µm, most preferably X90 ≥ 5 µm. Therefore preferred particle size distributions are such that 0.1 µm < X90 < 200 µm, particularly 0.1 µm < X90 ≤ 150 µm, more preferably 1 µm ≤ X90 ≤ 150 µm, even more preferably 5 µm ≤ X90 ≤ 100 µm. A preferred example of a particle size distribution of BI 1356 is such that X90 ≤ 50 µm or 10 µm ≤ X90 ≤ 50 µm. It can be found that a pharmaceutical composition comprising BI 1356 with a particle size distribution as indicated hereinbefore shows desired properties (e.g. with regard to dissolution, content uniformity, production, or the like). The indicated particle size properties are determined by laser-diffraction method, in particular low angle laser light scattering, i.e. Fraunhofer diffraction. Alternatively, the particle size properties can be also determined by microscopy (e.g. electron microscopy or scanning electron microscopy). The results of the particle size distribution determined by different techniques can be correlated with one another.

The present disclosure is not to be limited in scope by the specific embodiments described herein. Various modifications of the disclosure in addition to those described herein may become apparent to those skilled in the art from the present disclosure.

Further embodiments, features and advantages of the present disclosure may become apparent from the following examples. The following examples serve to illustrate, by way of example, the principles of the disclosure without restricting it.

### Examples

### 1. Mono-layer Tablet

The composition of mono-layer tablets for a DPP-4 inhibitor of this disclosure (BI 1356) + metformin HCI FDC (Film-coated Tablets) is shown in Table 1.

**Table 1: Composition of BI 1356 + Metformin HCI FDC Mono-layer Tablets**

| **Ingredient** | **Dose Strength (BI 1356 / metformin HCl), mg** | | | | | |
|---|---|---|---|---|---|---|
| | **2.5/500** | | **2.5/850** | | **2.5/1000** | |
| | [mg] | [%] | [mg] | [%] | [mg] | [%] |
| BI 1356 | 2,50 | 0,42 | 2,50 | 0,25 | 2,50 | 0,21 |
| Metformin Hydrochloride | 500,0 | 84,75 | 850,00 | 85,00 | 1000,00 | 84,75 |
| L-Arginine | 12,50 | 2,12 | 21,20 | 2,12 | 25,00 | 2,12 |
| Corn starch | 20,00 | 3,39 | 33,10 | 3,31 | 42,50 | 3,60 |
| Copovidone | 47,50 | 8,05 | 80,50 | 8,05 | 95,00 | 8,05 |
| Colloidal Anhydrous Silica | 2,50 | 0,42 | 4,20 | 0,42 | 5,00 | 0,42 |
| Magnesium stearate | 5,00 | 0,85 | 8,50 | 0,85 | 10,00 | 0.85 |
| Purified water* | 186** | | 315** | | 372** | |
| **Total Mass (tablet core)** | **590,00** | 100,00 | **1000,00** | 100,00 | **1180,00** | 100,00 |
| Hypromellose (5 mPa*s) | 6,00 | 50,00 | 8,00 | 50,00 | 9,00 | 50,00 |
| Propylene glycol | 0,60 | 5,00 | 0,80 | 5,00 | 0,90 | 5,00 |
| Talc | 2,88 | 18,50 | 2,96 | 18,50 | 4,455 | 18,50 |
| Titanium dioxide | 2,40 | 25,00 | 4,00 | 25,00 | 3,60 | 25,00 |
| Iron oxide, yellow | 0,12 | 1,25 | 0,20 | 1,25 | | |
| Iron oxide, red | | | 0.04 | 0,25 | 0.045 | 1,25 |
| Purified water** | 88** | | 117** | | 132** | |
| **Total Mass (film-coat)** | **12,00** | 100,00 | **16,00** | 100,00 | **18,00** | 100,00 |
| **Total Mass (coated** tablet) | **602,00** | | **1016,00** | | **1198,00** | |

| | | | | | | |
|---|---|---|---|---|---|---|
| ** Removed during processing, does not appear in final product | | | | | | |

### Manufacturing procedure (Mono-layer tablets):

DPP-4 inhibitor of this disclosure (e.g. BI 1356) + metformin HCI FDC mono-layer tablets are produced by a fluid-bed granulation process and a conventional tableting process with a rotary press. Optionally, metformin HCI and corn starch may be pre-treated by heating in a chamber of fluid-bed granulator to remove excessive HCI and/or impurity products before mixing with the active DPP-4 inhibitor ingredient. After the optional pre-treatment of metformin HCI and corn starch, the DPP-4 inhibitor is either added as powder and premixed before fluid-bed granulation is conducted by spraying of "Granulation Liquid" composed of copolyvidon (Kollidon VA64), L-arginine and purified water, or directly dispersed in the "granulation liquid". After finishing of fluid-bed granulation, the granulate is sieved with a suitable screen. The sieved granulate is blended with colloidal anhydrous silica (Aerosil 200) and magnesium stearate as a lubricant. The final mixture is compressed into tablets using a conventional rotary tablet press.

The tablet cores may be film-coated by an aqueous film-coating suspension, containing hypromellose as film-forming agent, propylene glycol as plasticizer, talc as glidant and the pigments yellow iron oxide and/or red iron oxide and titanium dioxide.

Narrative more specific description of the preferred manufacturing process for the mono-layer tablets:
a) Metformin HCI and corn starch are sieved using a screen with a mesh size of 0.5 to1 mm before dispensing.
b) L-arginine, BI 1356 and finally copolyvidon are dissolved resp. dispersed in purified water at ambient temperature with a propeller mixer to produce the "Granulation Liquid".
c) Metformin HCI and corn starch are sucked into a chamber of a suitable fluid-bed granulator and preheated up to a product temperature target of approx. 36°C.
d) Immediately after the product temperature target is reached, the "Granulation Liquid" is sprayed into the mixture for fluid-bed granulating under dry condition to avoid blocking during granulation.
e) At the end of spraying, the resultant granulate is dried at approx. 70 C inlet air temperature until the desired LOD value (i.e. 1 - 2 %) is reached.
f) The granulate is sieved using a screen with a mesh size of 0.5 to 1.0 mm.
g) The sieved granulate and colloidal anhydrous silica (Aerosil 200) are blended with a suitable blender. Aerosil 200 should be pre-sieved with a small portion of the sieved granulate through a 0.8 mm-screen before use.
h) Magnesium stearate is passed through a 0.8 mm sieve and added into the granulate. Subsequently the "Final Blend" is produced by final blending in the free-fall blender.
i) The "Final Blend" is compressed into tablets with a rotary press.
j) Titanium dioxide, propylene glycol and iron oxide (yellow, red or yellow and red) are dispersed in purified water with a high shear homo-mixer. Then, hypromellose and talc are added and dispersed with a homo-mixer and propeller mixer at ambient temperature to produce the "Coating Suspension".
k) The tablet cores are coated with the "Coating Suspension" to the target weight gain to produce the "Film-coated Tablets". The "Coating Suspension" should be stirred again before use and kept stirring slowly during the coating (spraying) process.

Narrative more specific description of an alternative manufacturing process for the mono-layer tablets:
a) Metformin HCI is sieved using a screen with a mesh size of 0.5 to1 mm before weighing.
b) L-arginine and copolyvidon are dissolved in purified water at ambient temperature with a propeller mixer to produce the "Granulation Liquid"
c) Metformin HCI and corn starch are heated in a chamber of fluid-bed granulator at 70 - 80 °C for more than 15 min until the product temperature reaches 60 °C
d) BI 1356 is added into the container, then blended with metformin HCI and corn starch in the fluid-bed granulator.
e) The "Granulation Liquid" is sprayed into the mixture for fluid-bed granulating under dry condition to avoid blocking during granulation.
f) At the end of spraying, the resultant granulate is dried at 70 - 80 °C until the desired LOD value (i.e. 1 - 2 %), in case the LOD is more than 2 %.
g) The granulate is sieved using a screen with a mesh size of 0.5 to 1.0 mm.
h) The sieved granulate and colloidal anhydrous silica (Aerosil 200) are blended with a suitable blender. Aerosil 200 should be sieved with a 0.5 mm-screen before use.
i) Magnesium stearate passed through a 0.5 mm sieve and added into the granulate. Subsequently the "Final Blend" is produced by final blending in the blender.
j) The "Final Blend" is compressed into tablets with a rotary press.
k) Hypromellose and propylene glycol are dissolved in purified water with a propeller mixer. Talc, titanium dioxide, and iron oxide (yellow, or yellow and red) are dispersed in purified water with a homo-mixer. The suspension is added into the hypromellose solution, then mixed with a propeller mixer at ambient temperature to produce the "Coating Suspension".
l) The tablet cores are coated with the "Coating Suspension" to the target weight gain to produce the "Film-coated Tablets". The "Coating Suspension" should be stirred again before use and kept stirring slowly during the coating (spraying) process.

### 2. Bi-layer Tablet

The composition of bi-layer tablets for a DPP-4 inhibitor of this disclosure (BI 1356) + metformin HCI FDC (Film-coated Tablets) is shown in Table 2.

**Table 2: Composition of BI 1356 + Metformin HCI FDC Bi-layer Tablets**

| **Ingredient** | **Dose Strength (BI 1356 / metformin HCl), mg** | | | | | |
|---|---|---|---|---|---|---|
| | **2.5/500** | | **2.5/850** | | **2.5/1000** | |
| | [mg] | [%] | [mg] | [%] | [mg] | [%] |
| ***BI 1356-portion:*** | *(450)* | *(100)* | *(450)* | *(100)* | *(450)* | *(100)* |
| BI 1356 | 2,50 | 0,556 | 2,50 | 0,556 | 2,50 | 0,556 |
| L-Arginine | 2,50 | 0,556 | 2,50 | 0,556 | 2,50 | 0,556 |
| D-mannitol | 334,75 | 74,39 | 334,75 | 74,39 | 334,75 | 74,39 |
| Pregelatinized starch | 45,00 | 10,00 | 45,00 | 10,00 | 45,00 | 10,00 |
| Corn starch | 45,00 | 10,00 | 45,00 | 10,00 | 45,00 | 10,00 |
| Copovidone | 13,50 | 3,00 | 13,50 | 3,00 | 13,50 | 3,00 |
| Magnesium stearate | 6,75 | 1,50 | 6,75 | 1,50 | 6,75 | 1,50 |
| ***Metformin HCl-portion:*** | *(570)* | *(100)* | *(969)* | *(100)* | *(1140)* | *(100)* |
| Metformin Hydrochloride | 500,0 | 87,72 | 850,00 | 87,72 | 1000,00 | 87,72 |
| Corn starch | 15,00 | 2,63 | 25,50 | 2,63 | 30,00 | 2,63 |
| Copovidone | 47,50 | 8,33 | 80,57 | 8,33 | 95,00 | 8,33 |
| Colloidal Anhydrous Silica | 2,50 | 0,44 | 4,25 | 0,44 | 5,00 | 0,44 |
| Magnesium stearate | 5,00 | 0,88 | 8,50 | 0,88 | 10,00 | 0,88 |
| **Total Mass (tablet core)** | **1020** | 100,00 | **1419** | 100,00 | **1590** | 100,00 |
| Hypromellose (5 mPa*s) | 8,00 | 50,00 | 9,50 | 50,00 | 11,00 | 50,00 |
| Propylene glycol | 0,80 | 5,00 | 0,95 | 5,00 | 1,10 | 5,00 |
| Talc | 2,96 | 18,50 | 3,515 | 18,50 | 4,07 | 18,50 |
| Titanium dioxide | 4,00 | 25,00 | 4,75 | 25,00 | 5,50 | 25,00 |
| Iron oxide, yellow | 0.20 | 1,25 | 0,2375 | 1,25 | 0,275 | 1,25 |
| Iron oxide, red | 0.04 | 0,25 | 0.0475 | 0,25 | 0.055 | 0,25 |
| **Total Mass (film-coat)** | **16,00** | 100,00 | **19,00** | 100,00 | **22,00** | 100,00 |
| **Total Mass (coated tablet)** | **1036** | 100,00 | **1438** | 100,00 | **1612** | 100,00 |

### Manufacturing procedure (Bi-layer tablets):

DPP-4 inhibitor of this disclosure (e.g. BI 1356) + metformin HCI FDC bi-layer tablets are produced by a high-shear wet granulation process (for DPP-4 inhibitor-granulate), a fluid-bed granulation process (for metformin HCI-granulate), and bi-layer tableting process with a multilayer rotary press.

**DPP-4 inhibitor-granulate:** By using a high-shear granulator the active DPP-4 inhibitor ingredient is pre-mixed with the diluents D-mannitol and pregelatinized starch. The mixture is moistened with granulating liquid, containing purified water and copovidone as a binder. After further mixing, drying and sieving, the dried granulate is blended with magnesium stearate as a lubricant.

Narrative more specific description of the manufacturing process for the BI 1356-granulate:
a. Copovidone and L-arginine are dissolved in purified water at ambient temperature to produce the Granulation Liquid.
b. BI 1356, mannitol and pregelatinized starch are blended in a suitable mixer, to produce the Pre-Mix.
c. The Pre-mix is moistened with the Granulation Liquid and subsequently granulated.
d. The moist granulate is sieved through a suitable sieve.
e. The granulate is dried at about 50 °C (maximum 60 °C) in a suitable dryer until the desired loss on drying value is obtained.
f. The dried granulate is sieved through a sieve with a mesh size of 1.0 mm.
g. Magnesium stearate is passed through a 1.0 mm sieve and added to the granulate. Subsequently the **"Final Blend A"** is produced by final blending in a suitable blender.

**Metformin HCl-granulate:** Metformin HCI and corn starch are pre-treated by heating in a chamber of fluid-bed granulator to remove excessive HCI and/or impurity products. After the pre-treatment of metformin HCI and corn starch, fluid-bed granulation is conducted by spraying of "Granulation Liquid" composed of copolyvidon (Kollidon VA64) and purified water. After finishing of fluid-bed granulation, the granulate is sieved with a suitable screen. The sieved granulate is blended with colloidal anhydrous silica (Aerosil 200) and magnesium stearate as a lubricant.

Narrative more specific description of the manufacturing process for the Metformin HCI-granulate:
a) Metformin HCI is sieved using a screen with a mesh size of 0.5 to1 mm before weighing.
b) Copolyvidon is dissolved in purified water at ambient temperature with a propeller mixer to produce the "Granulation Liquid"
c) Metformin HCI and corn starch are heated in a chamber of fluid-bed granulator at 70 - 80 °C for more than 15 min until the product temperature reaches 60 °C
d) The "Granulation Liquid" is sprayed into the mixture for fluid-bed granulating under dry condition to avoid blocking during granulation.
e) At the end of spraying, the resultant granulate is dried at 70 - 80 °C until the desired LOD value (i.e. 1 - 2 %), in case the LOD is more than 2 %.
f) The granulate is sieved using a screen with a mesh size of 0.5 to 1.0 mm.
g) The sieved granulate and colloidal anhydrous silica (Aerosil 200) are blended with a suitable blender. Aerosil 200 should be sieved with a 0.5 mm-screen before use.
h) Magnesium stearate passed through a 0.5 mm sieve and added into the granulate. Subsequently the **"Final Blend B"** is produced by final blending in the blender.

The **"Final Blend A"** and **"Final Blend B"** are compressed into bi-layer tablets using a multilayer rotary press. The tablet cores may be film-coated by an aqueous film-coating suspension, containing hypromellose as film-forming agent, propylene glycol as plasticizer, talc as glidant and the pigments yellow iron oxide and/or red iron oxide and titanium dioxide.

Narrative more specific description of the manufacturing process for the film-coating:
a) Hypromellose and propylene glycol are dissolved in purified water with a propeller mixer. Talc, titanium dioxide, and iron oxide (yellow, red or yellow and red) are dispersed in purified water with a homo-mixer. The suspension is added into the hypromellose solution, then mixed with a propeller mixer at ambient temperature to produce the "Coating Suspension".
b) The tablet cores are coated with the "Coating Suspension" to the target weight gain to produce the "Film-coated Tablets". The "Coating Suspension" should be stirred again before use and kept stirring slowly during the coating (spraying) process.

### 3. Tablet-in-Tablet or Bull's eye tablet

The composition of Tablet-in-Tablet or Bull's eye tablets for a DPP-4 inhibitor of this disclosure (BI 1356) + metformin HCI FDC (Film-coated Tablets) is shown in Table 3.

**Table 3: Composition of BI 1356 + Metformin HCI FDC Tablet-in-Tablet or Bull's Eye Tablets**

| **Ingredient** | **Dose Strength (BI 1356 / metformin HCl), mg** | | | | | |
|---|---|---|---|---|---|---|
| | **2.5/500** | | **2.5/850** | | **2.5/1000** | |
| | [mg] | [%] | [mg] | [%] | [mg] | [%] |
| ***BI 1356-portion:*** | *(45)* | *(100)* | *(45)* | *(100)* | *(45)* | *(100)* |
| Bl 1356 | 2,50 | 5,56 | 2,50 | 5,56 | 2,50 | 5,56 |
| L-Arginine | 1,00 | 2,22 | 1,00 | 2,22 | 1,00 | 2,22 |
| D-mannitol | 30,475 | 67,72 | 30,475 | 67,72 | 30,475 | 67,72 |
| Pregelatinized starch | 4,50 | 10,00 | 4,50 | 10,00 | 4,50 | 10,00 |
| Corn starch | 4,50 | 10,00 | 4,50 | 10,00 | 4,50 | 10,00 |
| Copovidone | 1,350 | 3,00 | 1,350 | 3,00 | 1,35 | 3,00 |
| Magnesium stearate | 0,675 | 1,50 | 0,675 | 1,50 | 6,75 | 1,50 |
| ***Metformin HCl-portion:*** | *(570)* | *(100)* | *(969)* | *(100)* | *(1140)* | *(100)* |
| Metformin Hydrochloride | 500,0 | 87,72 | 850,00 | 87,72 | 1000,00 | 87,72 |
| Corn starch | 15,00 | 2,63 | 25,50 | 2,63 | 30,00 | 2,63 |
| Copovidone | 47,50 | 8,33 | 80,57 | 8,33 | 95,00 | 8,33 |
| Colloidal Anhydrous Silica | 2,50 | 0,44 | 4,25 | 0,44 | 5,00 | 0,44 |
| Magnesium stearate | 5,00 | 0,88 | 8,50 | 0,88 | 10,00 | 0,88 |
| **Total Mass (tablet core)** | **615** | 100,00 | **1014** | 100,00 | **1185** | 100,00 |
| Hypromellose (5 mPa*s) | 6,00 | 50,00 | 8,00 | 50,00 | 9,00 | 50,00 |
| Propylene glycol | 0,60 | 5,00 | 0,80 | 5,00 | 0,90 | 5,00 |
| Talc | 2,22 | 18,50 | 2,96 | 18,50 | 3,33 | 18,50 |
| Titanium dioxide | 3,00 | 25,00 | 4,00 | 25,00 | 4,50 | 25,00 |
| Iron oxide, yellow | 0.15 | 1,25 | 0,20 | 1,25 | 0,225 | 1,25 |
| Iron oxide, red | 0.03 | 0,25 | 0.04 | 0,25 | 0.045 | 0,25 |
| **Total Mass (film-coat)** | **12,00** | 100,00 | **16,00** | 100,00 | **18,00** | 100,00 |
| **Total Mass (coated tablet)** | **627** | 100,00 | **1030** | 100,00 | **1203** | 100,00 |

### Manufacturing procedure (Tablet-in-Tablet or Bull's eye tablet):

DPP-4 inhibitor of this disclosure (e.g. Bl 1356) + metformin HCl FDC Tablet-in-Tablet or Bull's eye tablets are produced by a high-shear wet granulation process (for DPP-4 inhibitor-granulate), a rotary press (for DPP-4 inhibitor core-tablet), a fluid-bed granulation process (for metformin HCl-granulate), and press-coating process with a press-coater.

**DPP-4 inhibitor core-tablet:** By using a high-shear granulator the active DPP-4 inhibitor ingredient is pre-mixed with the diluents D-mannitol and pregelatinized starch. The mixture is moistened with granulating liquid, containing purified water and copovidone as a binder. After further mixing, drying and sieving, the dried granulate is blended with magnesium stearate as a lubricant.

Narrative more specific description of the manufacturing process for the Bl 1356 core-tablets:
a. Copovidone and L-arginine are dissolved in purified water at ambient temperature to produce the Granulation Liquid.
b. Bl 1356, mannitol and pregelatinized starch are blended in a suitable mixer, to produce the Pre-Mix.
c. The Pre-mix is moistened with the Granulation Liquid and subsequently granulated.
d. The moist granulate is sieved through a suitable sieve.
e. The granulate is dried at about 50 °C (maximum 60 °C) in a suitable dryer until the desired loss on drying value is obtained.
f. The dried granulate is sieved through a sieve with a mesh size of 1.0 mm.
g. Magnesium stearate is passed through a 1.0 mm sieve and added to the granulate. Subsequently the "Final Blend" is produced by final blending in a suitable blender.
h. "Final Blend" is compressed into **"Bl 1356 core-tablets"** with a rotary press.

**Metformin HCl-granulate:** Metformin HCl and corn starch are pre-treated by heating in a chamber of fluid-bed granulator to remove excessive HCI and/or impurity products. After the pre-treatment of metformin HCI and corn starch, fluid-bed granulation is conducted by spraying of "Granulation Liquid" composed of copolyvidon (Kollidon VA64) and purified water. After finishing of fluid-bed granulation, the granulate is sieved with a suitable screen. The sieved granulate is blended with colloidal anhydrous silica (Aerosil 200) and magnesium stearate as a lubricant.

Narrative more specific description of the manufacturing process for the Metformin HCI-granulate:
a) Metformin HCI is sieved using a screen with a mesh size of 0.5 to1 mm before weighing.
b) Copolyvidon is dissolved in purified water at ambient temperature with a propeller mixer to produce the "Granulation Liquid"
c) Metformin HCI and corn starch are heated in a chamber of fluid-bed granulator at 70 - 80 °C for more than 15 min until the product temperature reaches 60 °C
d) The "Granulation Liquid" is sprayed into the mixture for fluid-bed granulating under dry condition to avoid blocking during granulation.
e) At the end of spraying, the resultant granulate is dried at 70 - 80 °C until the desired LOD value (i.e. 1 - 2 %), in case the LOD is more than 2 %.
f) The granulate is sieved using a screen with a mesh size of 0.5 to 1.0 mm.
g) The sieved granulate and colloidal anhydrous silica (Aerosil 200) are blended with a suitable blender. Aerosil 200 should be sieved with a 0.5 mm-screen before use.
h) Magnesium stearate passed through a 0.5 mm sieve and added into the granulate. Subsequently **"Metformin HCl-granulate"** (Final Blend) is produced by final blending in the blender.

The **"DPP-4 inhibitor core-tablets"** and **"Metformin HCl-granulate"** are compressed into Tablet-in-Tablet or Bull's eye tablets using a press-coater. The difference between the Tablet-in-Tablet and Bull's eye tablet is the position of the core tablet.

Narrative more specific description of the manufacturing process for the Tablet-in-Tablet:
a) Fill a half of Metformin HCl-granulate in a die.
b) Place a Bl 1356 core-tablet on the surface of Metformin HCI-granulate.
c) Cover the core-tablet with second half of Metformin HCl-granulate, then compressed into the tablet (Tablet-in-Tablet).

Narrative more specific description of the manufacturing process for the Bull's eye tablets:
a) Fill Metformin HCl-granulate in a die.
b) Place the Bl 1356 core-tablet on the Metformin HCl-granulate in the die, then compressed into the tablet (Bull's eye tablet).

The tablets may be film-coated by an aqueous film-coating suspension, containing hypromellose as film-forming agent, propylene glycol as plasticizer, talc as glidant and the pigments yellow iron oxide and/or red iron oxide and titanium dioxide.

Narrative more specific description of the manufacturing process for the film-coating:
a) Hypromellose and propylene glycol are dissolved in purified water with a propeller mixer. Talc, titanium dioxide, and iron oxide (yellow, red or yellow and red) are dispersed in purified water with a homo-mixer. The suspension is added into the hypromellose solution, then mixed with a propeller mixer at ambient temperature to produce the "Coating Suspension".
b) The tablet cores are coated with the "Coating Suspension" to the target weight gain to produce the "Film-coated Tablets". The "Coating Suspension" should be stirred again before use and kept stirring slowly during the coating (spraying) process.

### 4. DPP-4 inhibitor-Drug Layering on Metformin HCl Tablet (film-coating for drug-loading)

The composition of a DPP-4 inhibitor of this disclosure (Bl 1356) + metformin HCl FDC (Film-coated Tablets) which are prepared by drug loading by film-coating on the Metformin HCl Tablet is shown in Table 4.

**Table 4: Composition of Bl 1356 + Metformin HCl FDC Bl 1356-Coating on Metformin HCl Tablet**

| **Ingredient** | **Dose Strength (Bl 1356 / metformin HCl), mg** | | | | | |
|---|---|---|---|---|---|---|
| | **2.5/500** | | **2.5/850** | | **2.5/1000** | |
| | [mg] | [%] | [mg] | [%] | [mg] | [%] |
| ***Metformin HCl-portion:*** | *(570)* | *(100)* | (969) | *(100)* | *(1140)* | *(100)* |
| Metformin Hydrochloride | 500,0 | 87,72 | 850,0 | 87,72 | 1000,0 | 87,72 |
| Corn starch | 15,0 | 2,63 | 25,5 | 2,63 | 30,0 | 2,63 |
| Copovidone | 47,5 | 8,33 | 80,57 | 8,33 | 95,0 | 8,33 |
| Colloidal Anhydrous Silica | 2,5 | 0,44 | 4,25 | 0,44 | 5,0 | 0,44 |
| Magnesium stearate | 5,0 | 0,88 | 8,5 | 0,88 | 10,0 | 0,88 |
| **Total Mass (tablet core)** | **570** | 100,00 | **969** | 100,00 | **1140** | 100,00 |
| ***Seal-coat*** (seal-coating): | *(12)* | *(100)* | *(16)* | *(100)* | *(18)* | *(100)* |
| Hypromellose (5 mPa*s) | 6,00 | 50,00 | 8,00 | 50,00 | 9,00 | 50,00 |
| Propylene glycol | 0,60 | 5,00 | 0,80 | 5,00 | 0,90 | 5,00 |
| Talc | 2,22 | 18,50 | 2,96 | 18,50 | 3,33 | 18,50 |
| Titanium dioxide | 3,00 | 25,00 | 4,00 | 25,00 | 4,50 | 25,00 |
| Iron oxide, yellow | 0.15 | 1,25 | 0,20 | 1,25 | 0,225 | 1,25 |
| Iron oxide, red | 0.03 | 0,25 | 0.04 | 0,25 | 0.045 | 0,25 |
| ***Drug-layer*** (drug-loading): | *(25)* | *(100)* | *(25)* | *(100)* | *(25)* | *(100)* |
| Bl 1356 | 2,50 | 10,00 | 2,50 | 10,00 | 2,50 | 10,00 |
| L-Arginine | 2,50 | 10,00 | 2,50 | 10,00 | 2,50 | 10,00 |
| Hypromellose (5 mPa*s) | 18,00 | 72,00 | 18,00 | 72,00 | 18,00 | 72,00 |
| Propylene glycol | 2,00 | 8,00 | 2,00 | 8,00 | 2,00 | 8,00 |
| ***Over-coat*** (over-coating): | *(12)* | *(100)* | *(16)* | *(100)* | *(18)* | *(100)* |
| Hypromellose (5 mPa*s) | 6,00 | 50,00 | 8,00 | 50,00 | 9,00 | 50,00 |
| Propylene glycol | 0,60 | 5,00 | 0,80 | 5,00 | 0,90 | 5,00 |
| Talc | 2,22 | 18,50 | 2,96 | 18,50 | 3,33 | 18,50 |
| Titanium dioxide | 3,00 | 25,00 | 4,00 | 25,00 | 4,50 | 25,00 |
| Iron oxide, yellow | 0.15 | 1,25 | 0,20 | 1,25 | 0,225 | 1,25 |
| Iron oxide, red | 0.03 | 0,25 | 0.04 | 0,25 | 0.045 | 0,25 |
| **Total Mass (film-coat)** | **49** | 100,00 | **57** | 100,00 | **61** | 100,00 |
| **Total Mass (coated tablet)** | **619** | 100,00 | **1026** | 100,00 | **1201** | 100,00 |

### Manufacturing procedure (DPP-4 inhibitor-drug layering by film-coating on Metformin HCl Tablet):

DPP-4 inhibitor (e.g. Bl 1356) + metformin HCI FDC with drug coating is produced by a fluid-bed granulation process, a conventional tableting process, and film-coating process with three steps: seal-coating, drug-loading and over-coating. The over-coating may be able to be skipped by combining with the drug-loading, if the stability is acceptable.

**Metformin HCl Tablets:** Metformin HCl and corn starch are pre-treated by heating in a chamber of fluid-bed granulator to remove excessive HCl and/or impurity products. After the pre-treatment of metformin HCl and corn starch, fluid-bed granulation is conducted by spraying of "Granulation Liquid" composed of copolyvidon (Kollidon VA64) and purified water. After finishing of fluid-bed granulation, the granulate is sieved with a suitable screen. The sieved granulate is blended with colloidal anhydrous silica (Aerosil 200) and magnesium stearate as a lubricant. The final blend is compressed into the tablets with a conventional rotary press.

Narrative more specific description of the manufacturing process for the Metformin HCl-granulate:
a) Metformin HCl is sieved using a screen with a mesh size of 0.5 to1 mm before weighing.
b) Copolyvidon is dissolved in purified water at ambient temperature with a propeller mixer to produce the "Granulation Liquid"
c) Metformin HCl and corn starch are heated in a chamber of fluid-bed granulator at 70 - 80 °C for more than 15 min until the product temperature reaches 60 °C
d) The "Granulation Liquid" is sprayed into the mixture for fluid-bed granulating under dry condition to avoid blocking during granulation.
e) At the end of spraying, the resultant granulate is dried at 70 - 80 °C until the desired LOD value (i.e. 1 - 2 %), in case the LOD is more than 2 %.
f) The granulate is sieved using a screen with a mesh size of 0.5 to 1.0 mm.
g) The sieved granulate and colloidal anhydrous silica (Aerosil 200) are blended with a suitable blender. Aerosil 200 should be sieved with a 0.5 mm-screen before use.
h) Magnesium stearate passed through a 0.5 mm sieve and added into the granulate. Subsequently "Final Blend" is produced by final blending in the blender.
i) The "Final Blend" is compressed into the tablets with a conventional rotary press.

**Film-coating:** The tablets are film-coated by (1) **seal**-**coating**: by an aqueous film-coating suspension, containing hypromellose as film-forming agent, propylene glycol as plasticizer, talc as glidant and the pigments yellow iron oxide and/or red iron oxide and titanium dioxide, (2) **drug**-**loading**: by an aqueous film-coating suspension, containing hypromellose as film-forming agent, propylene glycol as plasticizer, Bl 1356 as drug substance, and L-arginine as stabilizer, and (3) **over**-**coating**: by an aqueous film-coating suspension, containing hypromellose as film-forming agent, propylene glycol as plasticizer, talc as glidant and the pigments yellow iron oxide and/or red iron oxide and titanium dioxide,

Narrative more specific description of the manufacturing process for the film-coating with a coating machine:
a) Hypromellose and propylene glycol are dissolved in purified water with a propeller mixer. Talc, titanium dioxide, and iron oxide (yellow, red or yellow and red) are dispersed in purified water with a homo-mixer. The suspension is added into the hypromellose solution, then mixed with a propeller mixer at ambient temperature to produce the "**Coating Suspension**" for "seal-coating" and "over-coating".
b) Hypromellose, propylene glycol and L-arginine are dissolved in purified water with a propeller mixer. Bl 1356 (active drug) is added into the hypromellose solution, then dispersed with a propeller mixer at ambient temperature to produce the "**Drug Suspension**" for "drug-loading".
c) The Metformin HCI tablets are coated with the "Coating Suspension" to the target weight gain to form the "**seal**-**coat**". The "Coating Suspension" should be stirred again before use and kept stirring slowly during the coating (spraying) process.
d) Following the seal-coating, the "Drug Suspension" is applied to the surface of the Metformin HCl tablets to form the "**drug layer**" (drug loading). The "Drug Suspension" should be stirred again before use and kept stirring slowly during the coating (spraying) process. The coating end point can be determined by available PAT (Process Analysis Technology).
e) After drug loading the "Coating Suspension" is applied to the Bl 1356 drug-loaded tablets to form the "**over**-**coat**" and to produce the "Film-coated Tablets". The "Coating Suspension" should be stirred again before use and kept stirring slowly during the coating (spraying) process.

### Product Description:

The product description of Bl 1356 + Metformin HCl FDC mono-layer tablets (tablet core and film-coated tablets) is shown in Table 8 and Table 9, respectively.

**Table 8: Product Description of Bl 1356 + Metformin HCl FDC Mono-layer Tablets (Tablet Core)**

| **Items** | **Dose Strength (Bl 1356 / metformin HCl), mg** | | |
|---|---|---|---|
| | **2.5 / 500** | **2.5 / 850** | **2.5/1000** |
| Tablet shape | Oval, biconvex | Oval, biconvex | Oval, biconvex |
| Tablet size [mm] | 16.2 × 8.5 | 19.1 × 9.3 | 21.0 × 9.6 |
| Color | white | | |
| Weight | 590 | 1000 | 1180 |
| Thickness [mm], (Mean) | Approx. 5.8 | Approx. 7.3 | Approx. 7.6 |
| Crushing strength [N], (Mean) | ≥ 100, Approx. 140 | ≥ 150, Approx. 190 | ≥ 150, Approx. 200 |
| Disintegration time [min] | ≤ 15 | ≤ 15 | ≤ 15 |
| Friability [%] | ≤ 0,5 | ≤ 0,5 | ≤ 0,5 |

**Table 9: Product Description of Bl 1356 + Metformin HCl FDC Mono-layer Tablets (Coated)**

| **Items** | **Dose Strength (Bl 1356 / metformin HCl), mg** | | |
|---|---|---|---|
| | **2.5 / 500** | **2.5 / 850** | **2.5/1000** |
| Color | light yellow | light orange | light red |
| Weight | 602 | 1016 | 1198 |
| Thickness [mm], (Mean) | Approx. 5.9 | Approx. 7.4 | Approx. 7.7 |
| Crushing strength [N] (Mean) | ≥ 100, Approx. 180 | ≥ 150, Approx. 240 | ≥ 150, Approx. 250 |
| Disintegration time [min] | ≤ 15 | ≤ 15 | ≤ 15 |

### Stability Data:

Stability data of Bl 1356 + Metformin HCI FDC mono-layer tablets (tablet core) with or without L-arginine is shown in the following tables (over 2 weeks, 1 month and 3 months):
2.5+500 mg tablets + 12.5 mg arginine:

| Test parameter | Initial | 60°C glass bottle | | |
|---|---|---|---|---|
| | | 2 W | 1 M | 3 M |
| Degradation Bl 1356 (%) | | | | |
| Total | < 0.2 | < 0.2 | < 0.2 | < 0.2 |

2.5+500 mg tablets + 0 mg arginine:

| Test parameter | Initial | 60°C glass bottle | | |
|---|---|---|---|---|
| | | 2 W | 1 M | 3 M |
| Degradation Bl 1356 (%) | | | | |
| Total | < 0.2 | 1.1 | 2.9 | 8.5 |

2.5+1000 mg tablets + 25 mg arginine:

| Test parameter | Initial | 60°C glass bottle | | |
|---|---|---|---|---|
| | | 2 W | 1 M | 3 M |
| Degradation Bl 1356 (%) | | | | |
| Total | < 0.2 | < 0.2 | < 0.2 | 0.2 |

2.5+1000 mg tablets + 0 mg arginine:

| Test parameter | Initial | 60°C glass bottle | | |
|---|---|---|---|---|
| | | 2 W | 1 M | 3 M |
| Degradation Bl 1356 (%) | | | | |
| Total | < 0.2 | 1.9 | 4.7 | 13.6 |

### Bl 1356, a Potent and Selective DPP-4 Inhibitor, is safe and efficacious in Patients with Inadequately Controlled Type 2 Diabetes despite Metformin Therapy

Efficacy and safety of Bl 1356 (1, 5, or 10 mg qd), a potent and selective dipeptidyl peptidase-4 (DPP-4) inhibitor, was examined in inadequately controlled, metformin-treated (MET, ≥1 g daily) type 2 diabetic patients (T2DM; HbA1c at baseline 7.5-10.0%). Effects were compared to add-on of placebo (PBO) or of open label glimepiride (GLIM; 1 to 3 mg qd) in a 12-week randomized, double-blind study. Antidiabetic medication other than metformin was washed out for 6 weeks (34.7% of the patients).

The primary endpoint was change from baseline in HbA1c, adjusted for prior antidiabetic medication. 333 patients (mean baseline HbA1c 8.3%; fasting plasma glucose [FPG] 185 mg/dL) were randomized to Bl 1356, PBO or open-label GLIM. After 12 weeks, Bl 1356 treatment resulted in significant placebo corrected mean reductions in HbA1c (Bl 1356 1 mg, n=65, -0.39%; 5 mg, n=66, -0.75%; 10 mg, n=66, -0.73%). Patients receiving GLIM demonstrated a slightly greater mean PBO corrected reduction in HbA1c at Week 12 (n=64, -0.90%). Reductions in FPG from baseline to Week 12 with Bl 1356 were statistically significant (1 mg, -19 mg/dL; 5 mg, -35 mg/dL; 10 mg, -30 mg/dL). Hence, a dose-response relationship was demonstrated for HbA1c and FPG, reaching an effect plateau at 5 mg of Bl 1356. For this dose, >80% DPP-4 inhibition at trough in >80% of the patients at week 12 was achieved.

In total, 106 patients (43.1%) experienced adverse events (AEs) with similar incidences across all treatments. Most frequently reported episodes were nasopharyngitis (7.5%), diarrhoea (3.3%), and nausea (3.0%). Drug-related hypoglycaemia did not occur with Bl 1356 or PBO but in 3 patients receiving GLIM. Ten patients (3.7%) experienced serious AEs but none of these events were considered drug-related.

The addition of Bl 1356 to MET in patients with T2DM inadequately controlled on MET alone achieved clinically relevant and statistically significant reductions in HbA1c. Combination treatment with Bl 1356 1, 5, and 10 mg and MET was well tolerated and no case of hypoglycaemia was reported. The incidence of AEs was comparable with Bl 1356 and PBO.

### Efficacy and Safety of Linagliptin in Type 2 Diabetes Inadequately Controlled on Metformin Monotherapy

A multi-center, 24-week, randomized, placebo-controlled, double-blind, parallel group study examines the efficacy and safety of linagliptin (LI) administered as add-on therapy to metformin (MET) in type 2 diabetes mellitus (T2DM) hyperglycemic patients with insufficient glycemic control (HbA1c ≥7 to ≤10.0% for patients previously treated only with metformin, or ≥6.5 to ≤9.0% for patients previously treated with additional oral antihyperglycemic drugs). Subjects who enter the screening period discontinue previous antidiabetic medication other than MET (≥1500 mg/day) for 6 weeks (including a placebo (PBO) run-in period during the last 2 weeks) prior to randomization to LI (n=524) or PBO (n=177). Mean baseline characteristics and demographics (HbA1c, 8.1%; fasting plasma glucose [FPG], 168.8 mg/dL; age, 56.5 yrs; BMI, 29.9 kg/m2) are similar between groups. The primary endpoint is the change from baseline HbA1c after 24 weeks of treatment, evaluated with an analysis of covariance (ANCOVA) adjusted for baseline HbA1c and prior antidiabetic medication. After 24 weeks of treatment, the adjusted mean treatment difference between LI + MET and PBO+MET is -0.64% (p<0.0001) in favor of LI+MET for change in HbA1c (%). Patients with a baseline HbA1c of ≥7.0% who receive LI+MET are more likely to achieve an HbA1c ≤7.0% relative to those receiving placebo+MET (26.2% vs. 9.2%, respectively; odds ratio, 4.4; p=0.0001). At week 24 LI+MET is superior to PBO+MET in reducing the mean fasting plasma glucose (FPG) from baseline (-21.1 mg/dL; p<0.0001). At study-end, 2hr post-prandial glucose (PPG) analyzed in meal tolerance tests shows a significantly greater (p<0.0001) mean reduction from baseline for the LI+MET treated (-67.1 mg/dL) versus the PBO+MET group. The proportion of patients reporting at least one adverse event (AE) is comparable within the LI+MET and PBO+MET groups (52.8% and 55.4%, respectively). Hypoglycemia is rare, occurring in 5 PBO+MET patients (2.8%) and 3 LI+MET patients (0.6%), all episodes being of mild intensity. The change in the body weight from baseline to 24 weeks is similar between the 2 treatment groups (-0.5 kg PBO+MET; -0.4 kg LI+MET). Conclusion, linagliptin 5 mg qd as add-on therapy in patients with T2DM inadequately controlled on metformin is well tolerated and produces significant and clinically meaningful improvements in glycemic control (reductions in HbA1c, FPG and 2h PPG without weight gain). Linagliptin as add-on therapy to metformin in patients with T2DM and insufficient glycemic control is well tolerated with the incidence of adverse events comparable to placebo.

### Linagliptin improves Glycemic Control in Asian Type 2 Diabetes Patients Inadequately Controlled on Metformin Monotherapy

A multi-center, 24-wk, placebo (PBO)-controlled study examines efficacy and safety of the DPP-4 inhibitor linagliptin (LI) (5 mg qd) as add-on to metformin (MET) therapy in T2DM patients (pts) with insufficient glycemic control. All pts (HbA1c ≥7.0 to ≤10.0% if previously treated with MET, or ≥6.5 to 9.0% if treated with another oral antihyperglycemic drug [OAD]) discontinue OADs other than MET (≥1500 mg/day) for 6 wks (including a PBO run-in period during the last 2 wks) before randomization to LI+MET (n=524; of which n=111 were Asian) or PBO+MET (n=177; n=32 Asian). At wk 24, the PBO-adjusted mean treatment difference in HbA1c in the Asian pts is -0.86% (P<0.0001) in favor of LI+MET. The difference in the adjusted mean change from baseline (BL) at wk 24 for fasting plasma glucose is -21 mg/dL for the full analysis set (FAS) pts on LI+MET (P<0.0001). 2h post-prandial glucose (PPG) analyzed in meal tolerance tests, shows a reduction from BL of-67 mg/dL for LI+MET FAS pts (P<0.0001) at wk 24. Numbers of pts reporting at least 1 adverse event are comparable for the LI (52.8%) and PBO (55.4%) groups (treated set). Hypoglycemia is rare (0.6% of LI pts; 2.8% of PBO pts). Linagliptin as add-on therapy in pts with T2DM inadequately controlled on MET delivers significant and clinically meaningful reductions in HbA1c, FPG and 2hPPG without weight gain. In the Asian patient subgroup Linagliptin significantly improves glycemic control.

### Linagliptin improves Glycemic Control in Asian Type 2 Diabetes Patients Inadequately Controlled on Metformin and Sulfonylurea Dual Combination Therapy

A multi-center, 24-wk, placebo (PBO)-controlled study examines the efficacy and safety of the oral DPP-4 inhibitor linagliptin (LI; 5 mg qd) in type 2 diabetes (T2DM) patients (pts) with insufficient glycemic control (HbA1c ≥7.0 to ≤10.0%) on the combination of metformin (MET) plus sulfonylurea (SU). All pts have a 2-wk PBO run-in before randomization to LI+MET+SU (n=793; of which n=397 are Asian) or PBO+MET+SU (n=265; n=141 Asian). Pts from China (193, 18%) and Korea (174, 16%) make up the highest numbers of Asian race pts enrolled. At wk 24, the PBO-adjusted mean treatment difference in HbA1c in the Asian pts is -0.71% (P<0.0001) in favor of LI+MET+SU. For the full analysis set (FAS), the adjusted mean difference between LI and PBO for the change in fasting plasma glucose (FPG) from baseline at wk 24 is -13 mg/dL (P<0.0001). Measures of β-cell function (HOMA-%B) and insulin resistance (HOMA-IR) improve with LI vs. PBO pts in the FAS (P<0.05). Reported severe adverse events (AEs) are low for LI+MET+SU (2.4%) and PBO+MET+SU (1.5%) pts (treated set). No significant changes in weight are noted (FAS). Linagliptin add-on to MET and SU combination therapy has a favorable safety and tolerability profile and is weight neutral. This combination significantly improves glycemic control in T2DM pts, including Asian pts. A low risk for hypoglycemia should be considered when linagliptin is indicated as add-on to pre-existing sulfonylurea therapy. Linagliptin provides an additional option prior to insulin therapy in patients for whom glycemia is insufficiently controlled with metformin plus a sulfonylurea agent.

## Claims

1. A pharmaceutical composition comprising or made from
a DPP-4 inhibitor which is 1-[(4-methyl-quinazolin-2-yl)methyl]-3-methyl-7-(2-butyn-1-yl)-8-(3-(R)-amino-piperidin-1-yl)-xanthine,
metformin,
a stabilizer which is a basic amino acid,
and one or more pharmaceutical excipients;
for use in treating and/or preventing (including slowing the progression and/or delaying the onset) of metabolic diseases, especially type 2 diabetes mellitus and conditions related thereto (e.g. diabetic complications),
either in type 2 diabetes patients who have not been previously treated with an antihyperglycemic agent,
or in type 2 diabetes patients with insufficient glycemic control despite therapy with one or two conventional antihyperglycemic agents selected from metformin, sulphonylureas, thiazolidinediones (e.g. pioglitazone), glinides, alpha-glucosidase blockers, GLP-1 or GLP-1 analogues, and insulin or insulin analogues;
optionally in combination with one or more other active substances.

2. A pharmaceutical composition comprising or made from
a DPP-4 inhibitor which is 1-[(4-methyl-quinazolin-2-yl)methyl]-3-methyl-7-(2-butyn-1-yl)-8-(3-(R)-amino-piperidin-1-yl)-xanthine,
metformin,
a stabilizer which is a basic amino acid ,
and one or more pharmaceutical excipients;
for use in one or more of the following methods:
- preventing, slowing the progression of, delaying the onset of or treating a metabolic disorder or disease, such as e.g. type 1 diabetes mellitus, type 2 diabetes mellitus, impaired glucose tolerance (IGT), impaired fasting blood glucose (IFG), hyperglycemia, postprandial hyperglycemia, overweight, obesity, dyslipidemia, hyperlipidemia, hypercholesterolemia, hypertension, atherosclerosis, endothelial dysfunction, osteoporosis, chronic systemic inflammation, non-alcoholic fatty liver disease (NAFLD), retinopathy, neuropathy, nephropathy and/or metabolic syndrome;
- improving glycemic control and/or for reducing of fasting plasma glucose, of postprandial plasma glucose and/or of glycosylated hemoglobin HbA1c;
- preventing, slowing, delaying the onset of or reversing progression from impaired glucose tolerance (IGT), impaired fasting blood glucose (IFG), insulin resistance and/or from metabolic syndrome to type 2 diabetes mellitus;
- preventing, reducing the risk of, slowing the progression of, delaying the onset of or treating of complications of diabetes mellitus such as micro- and macrovascular diseases, such as nephropathy, micro- or macroalbuminuria, proteinuria, retinopathy, cataracts, neuropathy, learning or memory impairment, neurodegenerative or cognitive disorders, cardio- or cerebrovascular diseases, tissue ischaemia, diabetic foot or ulcus, atherosclerosis, hypertension, endothelial dysfunction, myocardial infarction, acute coronary syndrome, unstable angina pectoris, stable angina pectoris, peripheral arterial occlusive disease, cardiomyopathy, heart failure, heart rhythm disorders, vascular restenosis, and/or stroke;
- reducing body weight or preventing an increase in body weight or facilitating a reduction in body weight;
- preventing, slowing the progression of, delaying the onset of or treating the degeneration of pancreatic beta cells and/or the decline of the functionality of pancreatic beta cells and/or for improving and/or restoring the functionality of pancreatic beta cells and/or stimulating and/or restoring the functionality of pancreatic insulin secretion;
- preventing, slowing the progression of, delaying the onset of or treating non alcoholic fatty liver disease (NAFLD) including hepatic steatosis, non-alcoholic steatohepatitis (NASH) and/or liver fibrosis;
- preventing, slowing the progression of, delaying the onset of or treating type 2 diabetes with primary or secondary failure to conventional (oral or non-oral) antihyperglycemic mono- or combination therapy;
- achieving a reduction in the dose of conventional antihyperglycemic medication required for adequate therapeutic effect;
- reducing the risk for adverse effects associated with conventional antihyperglycemic medication; and/or
- maintaining and/or improving the insulin sensitivity and/or for treating or preventing hyperinsulinemia and/or insulin resistance;
either in a type 2 diabetes patient who has not been previously treated with an antihyperglycemic agent or in a type 2 diabetes patient with insufficient glycemic control despite therapy with one or two conventional antihyperglycemic agents selected from metformin, sulphonylureas, thiazolidinediones, glinides, alpha-glucosidase blockers, GLP-1 or GLP-1 analogues, and insulin or insulin analogues;
optionally in combination with one or more other active substances.

3. The pharmaceutical composition for use according to claim 1 or 2 which is a solid pharmaceutical composition (e.g. oral tablet) comprising or made from 1-[(4-methyl-quinazolin-2-yl)methyl]-3-methyl-7-(2-butyn-1-yl)-8-(3-(R)-amino-piperidin-1-yl)-xanthine,
metformin hydrochloride,
L-arginine (particularly as stabilizer),
and one or more fillers, one or more binders, one or more glidants and/or one or more lubricants.

4. The pharmaceutical composition for use according to claim 1, 2 or 3, wherein the type 2 diabetes patients have not been previously treated with an antihyperglycemic agent (e.g. drug-naive patients).

5. The pharmaceutical composition for use according to claim 1, 2 or 3, wherein the type 2 diabetes patients are with insufficient glycemic control on metformin, with or without a thiazolidinedione (e.g. pioglitazone) or a sulphonylurea.

6. The pharmaceutical composition for use according to claim 1, 2 or 3, wherein the type 2 diabetes patients are with insufficient glycemic control despite mono-therapy with metformin.

7. The pharmaceutical composition for use according to claim 1, 2 or 3, in combination with a thiazolidinedione (e.g. pioglitazone), wherein the type 2 diabetes patients having insufficient glycemic control despite dual combination therapy with metformin and a thiazolidinedione (e.g. pioglitazone).

8. The pharmaceutical composition for use according to claim 1, 2 or 3, in combination with a sulphonylurea, wherein the type 2 diabetes patients having insufficient glycemic control despite dual combination therapy with metformin and a sulphonylurea.

9. The pharmaceutical composition for use according to any of claims 1 to 8, wherein the metabolic disease is selected from type 1 diabetes mellitus, type 2 diabetes mellitus, impaired glucose tolerance (IGT), impaired fasting blood glucose (IFG), hyperglycemia, postprandial hyperglycemia, overweight, obesity, dyslipidemia, hyperlipidemia, hypercholesterolemia, hypertension, atherosclerosis, endothelial dysfunction, osteoporosis, chronic systemic inflammation, non-alcoholic fatty liver disease (NAFLD), retinopathy, neuropathy, nephropathy, and metabolic syndrome.

10. The pharmaceutical composition for use according to any of claims 1 to 8, wherein the diabetic complication is selected from micro- and macrovascular diseases, such as nephropathy, micro- or macroalbuminuria, proteinuria, retinopathy, cataracts, neuropathy, learning or memory impairment, neurodegenerative or cognitive disorders, cardio- or cerebrovascular diseases, tissue ischaemia, diabetic foot or ulcus, atherosclerosis, hypertension, endothelial dysfunction, myocardial infarction, acute coronary syndrome, unstable angina pectoris, stable angina pectoris, peripheral arterial occlusive disease, cardiomyopathy, heart failure, heart rhythm disorders, vascular restenosis, and stroke.

11. The pharmaceutical composition for use according to any of claims 1 to 8, wherein the metabolic disease is type 2 diabetes mellitus.

12. The composition for use according to claim 3, which is for use in improving glycemic control in type 2 diabetes patients who have not been previously treated with an antihyperglycemic agent (e.g. as first line therapy).

13. The composition for use according to claim 3, which is for use in improving glycemic control in type 2 diabetes patients with insufficient glycemic control despite mono-therapy with metformin (e.g. as second line therapy).

14. The composition for use according to claim 3 in combination with a thiazolidinedione (e.g. pioglitazone), which is for use in improving glycemic control in type 2 diabetes patients with insufficient glycemic control despite dual combination therapy with metformin and a thiazolidinedione (e.g. as third line therapy).

15. The composition for use according to claim 3 in combination with a sulphonylurea, which is for use in improving glycemic control in type 2 diabetes patients with insufficient glycemic control despite dual combination therapy with metformin and a sulphonylurea (e.g. as third line therapy).

16. The pharmaceutical composition for use according to any of claims 3, 12 to 15, wherein
the DPP-4 inhibitor is present in a dosage strength
of 2.5 mg, preferably for twice daily oral administration; or
of 5 mg, preferably for once daily oral administration.

17. The pharmaceutical composition for use according to any of claims 3, 12 to 16, wherein metformin hydrochloride is present in a dosage strength
of 500 mg, 850 mg or 1000 mg, preferably in form of immediate release metformin, preferably for twice daily oral administration;
or of 500 mg, 750 mg, 1000 mg or 1500 mg, preferably in form of extended release metformin, preferably for once daily oral administration.

18. The pharmaceutical composition for use according to any of claims 3, 12 to 17, wherein the DPP-4 inhibitor is present in a dosage strength of 2.5 mg and metformin hydrochloride is present in a dosage strength of 500 mg, 850 mg or 1000 mg.

19. The pharmaceutical composition for use according to any of claims 3, 12 to 18, which is for twice daily oral administration to the patient.

20. The pharmaceutical composition for use according to any of claims 3, 12 to 19, wherein L-arginine is present from about 1 mg to about 50 mg, preferably from about 1 mg to about 25 mg.

21. The pharmaceutical composition for use according to any of claims 3, 12 to 20, wherein the DPP-4 inhibitor and L-arginine are present in a weight ratio from about 1:20 to about 10:1, or from about 1:15 to about 10:1, preferably from about 1:10 to about 10:1.

22. The pharmaceutical composition for use according to any of claims 3, 12 to 21, wherein the excipients are selected from the group consisting of one or more fillers including D-mannitol, corn starch and pregelatinized starch; a binder including copovidone; a lubricant including magnesium stearate; and a glidant including colloidal anhydrous silica.

23. The pharmaceutical composition for use according to any of claims 3, 12 to 22 comprising copovidone as binder.

24. The pharmaceutical composition for use according to claim 23 further comprising one or more of the following: the filler corn starch, the lubricant magnesium stearate, and the glidant colloidal anhydrous silica.

25. The pharmaceutical composition for use according to any of the preceding claims in the dosage form of a tablet.

26. The pharmaceutical composition for use according to claim 25, wherein the tablet is selected from a mono-layer tablet, a bi-layer tablet, a press-coated tablet (including a tablet-in-tablet and a Bull's eye tablet), and a tablet which is film-coated for drug-loading.

27. The pharmaceutical composition for use according to claim 25 or 26, wherein the tablet comprises a film-coat.

28. The pharmaceutical composition for use according to claim 27, wherein the film-coat comprises a film-coating agent, such as e.g. hypromellose; a plasticizer, such as e.g. propylene glycol; optionally a glidant, such as e.g. talc, and optionally one or more pigments, such as e.g. titanium dioxide, iron oxide red and/or iron oxide yellow.

29. The pharmaceutical composition for use according to any of the preceding claims, which is an immediate release dosage form, **characterized in that** in a dissolution test after 45 minutes at least 75 % by weight of each of the active ingredients is dissolved.

30. The pharmaceutical composition for use according to any of the preceding claims, wherein 1-[(4-methyl-quinazolin-2-yl)methyl]-3-methyl-7-(2-butyn-1-yl)-8-(3-(R)-amino-piperidin-1-yl)-xanthine has a particle size distribution of X90 < 200 µm.

31. The pharmaceutical composition for use according to claim 3 comprising the active ingredients in an amount of:
(1) 2.5 mg of 1-[(4-methyl-quinazolin-2-yl)methyl]-3-methyl-7-(2-butyn-1-yl)-8-(3-(R)-amino-piperidin-1-yl)-xanthine free base, and 500 mg metformin hydrochloride; or
(2) 2.5 mg of 1-[(4-methyl-quinazolin-2-yl)methyl]-3-methyl-7-(2-butyn-1-yl)-8-(3-(R)-amino-piperidin-1-yl)-xanthine free base, and 850 mg metformin hydrochloride; or
(3) 2.5 mg of 1-[(4-methyl-quinazolin-2-yl)methyl]-3-methyl-7-(2-butyn-1-yl)-8-(3-(R)-amino-piperidin-1-yl)-xanthine free base, and 1000 mg metformin hydrochloride.

## Patentansprüche

1. Pharmazeutische Zusammensetzung, enthaltend oder hergestellt aus
einem DPP-4-Hemmer, der 1-[(4-Methyl-quinazolin-2-yl)methyl]-3-methyl-7-(2-butyn-1-yl)-8-(3-(R)-amino-piperidin-1-yl)-xanthin ist,
Metformin,
einem Stabilisator, der eine basische Aminosäure ist,
und einem oder mehreren pharmazeutischen Hilfsstoffen;
zur Verwendung bei der Behandlung und/oder Vorbeugung (die Verlangsamung des Fortschreitens und/oder die Verzögerung des Ausbruchs beinhaltend) von Stoffwechselerkrankungen, insbesondere Diabetes mellitus Typ 2 und damit zusammenhängende Erkrankungen (z. B. diabetische Komplikationen),
entweder bei Patienten mit Diabetes Typ 2, die zuvor nicht mit einem Antidiabetikum behandelt wurden,
oder bei Patienten mit Diabetes Typ 2 mit unzureichender Blutzuckereinstellung trotz Therapie mit einem oder zwei herkömmlichen Antidiabetika, ausgewählt aus Metformin, Sulfonylharnstoffen, Thiazolidindionen (z. B. Pioglitazon), Gliniden, Alpha-Glucosidase-Blockern, GLP-1 oder GLP-1-Analoga und Insulin oder Insulinanaloga;
optional in Kombination mit einem oder mehreren anderen Wirkstoffen.

2. Pharmazeutische Zusammensetzung, enthaltend oder hergestellt aus
einem DPP-4-Hemmer, der 1-[(4-Methyl-quinazolin-2-yl)methyl]-3-methyl-7-(2-butyn-1-yl)-8-(3-(R)-amino-piperidin-1-yl)-xanthin ist,
Metformin, einem Stabilisator, der eine basische Aminosäure ist,
und einem oder mehreren pharmazeutischen Hilfsstoffen;
zur Verwendung in einem oder mehreren der folgenden Verfahren:
- die Vorbeugung, die Verlangsamung des Fortschreitens von, die Verzögerung des Ausbruchs oder die Behandlung einer Stoffwechselstörung oder Krankheit, wie etwa z. B. Diabetes mellitus Typ 1, Diabetes mellitus Typ 2, gestörte Glukosetoleranz (IGT), gestörter Nüchternblutzucker (IFG), Hyperglykämie, postprandiale Hyperglykämie, Übergewicht, Adipositas, Dyslipidämie, Hyperlipidämie, Hypercholesterinämie, Bluthochdruck, Atheriosklerose, Endotheldysfunktion, Osteoporose, chronische systemische Entzündung, nichtalkoholische Fettlebererkrankung (NAFLD), Retinopathie, Neuropathie, Nephropathie und/oder metabolisches Syndrom;
- die Verbesserung der Blutzuckereinstellung und/oder zur Senkung der Nüchtern-Plasmaglukose, der postprandialen Plasmaglukose und/oder von glykosyliertem Hämoglobin HbA1c;
- die Verhinderung, die Verlangsamung, die Verzögerung des Beginns oder die Umkehrung des Fortschreitens von gestörter Glukosetoleranz (IGT), gestörtem Nüchternblutzucker (IFG), Insulinresistenz und/oder vom metabolischen Syndrom bis zum Diabetes mellitus Typ 2;
- die Vorbeugung, die Verringerung des Risikos, die Verlangsamung des Fortschreitens, die Verzögerung des Ausbruchs oder die Behandlung von Komplikationen von Diabetes mellitus wie etwa mikro- und makrovaskuläre Erkrankungen wie etwa Nephropathie, Mikro- oder Makroalbuminurie, Proteinurie, Retinopathie, Katarakte, Neuropathie, Lern- oder Gedächtnisstörungen, neurodegenerative oder kognitive Störungen, kardio- oder zerebrovaskuläre Erkrankungen, Gewebeischämie, diabetischer Fuß oder Ulkus, Atheriosklerose, Bluthochdruck, Endotheldysfunktion, Myokardinfarkt, akutes Koronarsyndrom, instabile Angina pectoris, stabile Angina pectoris, periphere arterielle Verschlusskrankheit, Kardiomyopathie, Herzinsuffizienz, Herzrhythmusstörungen, vaskuläre Restenose, und/oder Schlaganfall;
- die Verringerung des Körpergewichts oder die Verhinderung einer Zunahme des Körpergewichts oder die Erleichterung einer Verringerung des Körpergewichts;
- die Vorbeugung, die Verlangsamung des Fortschreitens, die Verzögerung des Einsetzens oder die Behandlung der Rückbildung von Betazellen der Bauchspeicheldrüse und/oder der Rückgang der Funktion von Betazellen der Bauchspeicheldrüse und/oder zur Verbesserung und/oder Wiederherstellung der Funktion von Betazellen der Bauchspeicheldrüse und/oder das Anregen und/oder die Wiederherstellung der Funktion der Insulinsekretion der Bauchspeicheldrüse;
- die Vorbeugung, die Verlangsamung des Fortschreitens, die Verzögerung des Ausbruchs oder die Behandlung einer nichtalkoholischen Fettlebererkrankung (NAFLD), was hepatische Steatose, nichtalkoholische Steatohepatitis (NASH) und/oder Leberfibrose beinhaltet;
- die Vorbeugung, die Verlangsamung des Fortschreitens, die Verzögerung des Ausbruchs oder die Behandlung von Diabetes Typ 2 mit Primär- oder Sekundärversagen einer herkömmlichen (oralen oder nicht-oralen) antihyperglykämischen Mono- oder Kombinationstherapie;
- das Erzielen einer Verringerung der Dosis herkömmlicher antihyperglykämischer Medikamente, die für eine angemessene therapeutische Wirkung erforderlich ist;
- die Verringerung der Gefahr von Nebenwirkungen im Zusammenhang mit herkömmlichen antihyperglykämischen Medikamenten; und/oder
- das Beibehalten und/oder das Verbessern der Insulinempfindlichkeit und/oder zur Behandlung oder Vorbeugung von Hyperinsulinämie und/oder Insulinresistenz;
entweder bei einem Patienten mit Diabetes Typ 2, der zuvor nicht mit einem Antidiabetikum behandelt wurde, oder bei einem Patienten mit Diabetes Typ 2 mit unzureichender Blutzuckereinstellung trotz Therapie mit einem oder zwei herkömmlichen Antidiabetika, ausgewählt aus Metformin, Sulfonylharnstoffen, Thiazolidindionen, Gliniden, Alpha-Glucosidase-Blockern, GLP-1 oder GLP-1-Analoga und Insulin oder Insulinanaloga;
optional in Kombination mit einem oder mehreren anderen Wirkstoffen.

3. Pharmazeutische Zusammensetzung zur Verwendung nach Anspruch 1 oder 2, die eine feste pharmazeutische Zusammensetzung (z. B. Tablette zum Einnehmen) ist, die Folgendes umfasst oder daraus hergestellt ist:
1-[(4-Methyl-quinazolin-2-yl)methyl]-3-methyl-7-(2-butyn-1-yl)-8-(3-(R)-amino-piperidin-1-yl)-xanthin,
Metforminhydrochlorid,
L-Arginin (insbesondere als Stabilisator),
und ein oder mehrere Füllstoffe, ein oder mehrere Bindemittel, ein oder mehrere Gleitmittel und/oder ein oder mehrere Schmiermittel.

4. Pharmazeutische Zusammensetzung zur Verwendung nach Anspruch 1, 2 oder 3, wobei die Patienten mit Diabetes Typ 2 zuvor nicht mit einem Antidiabetikum behandelt wurden (z. B. arzneimittelnaive Patienten).

5. Pharmazeutische Zusammensetzung zur Verwendung nach Anspruch 1, 2 oder 3, wobei die Patienten mit Diabetes Typ 2 eine unzureichende Blutzuckereinstellung auf Metformin mit oder ohne Thiazolidindion (z. B. Pioglitazon) oder einem Sulfonylharnstoff aufweisen.

6. Pharmazeutische Zusammensetzung zur Verwendung nach Anspruch 1, 2 oder 3, wobei die Patienten mit Diabetes Typ 2 trotz Monotherapie mit Metformin eine unzureichende Blutzuckereinstellung aufweisen.

7. Pharmazeutische Zusammensetzung zur Verwendung nach Anspruch 1, 2 oder 3 in Kombination mit einem Thiazolidindion (z. B. Pioglitazon), wobei die Patienten mit Diabetes Typ 2 trotz dualer Kombinationstherapie mit Metformin und einem Thiazolidindion (z. B. Pioglitazon) eine unzureichende Blutzuckereinstellung aufweisen.

8. Pharmazeutische Zusammensetzung zur Verwendung nach Anspruch 1, 2 oder 3 in Kombination mit einem Sulfonylharnstoff, wobei die Patienten mit Diabetes Typ 2 trotz dualer Kombinationstherapie mit Metformin und einem Sulfonylharnstoff eine unzureichende Blutzuckereinstellung aufweisen.

9. Pharmazeutische Zusammensetzung zur Verwendung nach einem der Ansprüche 1 bis 8, wobei die Stoffwechselerkrankung ausgewählt ist aus Diabetes mellitus Typ 1, Diabetes mellitus Typ 2, gestörter Glukosetoleranz (IGT), gestörtem Nüchternblutzucker (IFG), Hyperglykämie, postprandialer Hyperglykämie, Übergewicht, Adipositas, Dyslipidämie, Hyperlipidämie, Hypercholesterinämie, Bluthochdruck, Atheriosklerose, Endotheldysfunktion, Osteoporose, chronische systemische Entzündung, nichtalkoholische Fettlebererkrankung (NAFLD), Retinopathie, Neuropathie, Nephropathie und metabolischem Syndrom.

10. Pharmazeutische Zusammensetzung zur Verwendung nach einem der Ansprüche 1 bis 8, wobei die diabetische Komplikation ausgewählt ist aus mikro- und makrovaskulären Erkrankungen, wie etwa Nephropathie, Mikro- oder Makroalbuminurie, Proteinurie, Retinopathie, Katarakte, Neuropathie, Lern- oder Gedächtnisstörung, neurodegenerative oder kognitive Störungen, kardio- oder zerebrovaskuläre Erkrankungen, Gewebeischämie, diabetischer Fuß oder Ulkus, Atheriosklerose, Bluthochdruck, Endotheldysfunktion, Myokardinfarkt, akutes Koronarsyndrom, instabile Angina pectoris, stabile Angina pectoris, periphere arterielle Verschlusskrankheit, Kardiomyopathie, Herzinsuffizienz, Herzrhythmusstörungen, vaskuläre Restenose und Schlaganfall.

11. Pharmazeutische Zusammensetzung zur Verwendung nach einem der Ansprüche 1 bis 8, wobei die Stoffwechselerkrankung Diabetes mellitus Typ 2 ist.

12. Zusammensetzung zur Verwendung nach Anspruch 3, die zur Verwendung bei der Verbesserung der Blutzuckereinstellung bei Patienten mit Diabetes Typ 2 bestimmt ist, die zuvor nicht mit einem Antidiabetikum (z. B. als Erstlinientherapie) behandelt wurden.

13. Zusammensetzung zur Verwendung nach Anspruch 3, die zur Verwendung bei der Verbesserung der Blutzuckereinstellung bei Patienten mit Diabetes Typ 2 mit unzureichender Blutzuckereinstellung trotz Monotherapie mit Metformin (z. B. als Zweitlinientherapie) bestimmt ist.

14. Zusammensetzung zur Verwendung nach Anspruch 3 in Kombination mit einem Thiazolidindion (z. B. Pioglitazon), die zur Verwendung bei der Verbesserung der Blutzuckereinstellung bei Patienten mit Diabetes Typ 2 mit unzureichender Blutzuckereinstellung trotz dualer Kombinationstherapie mit Metformin und einem Thiazolidindion (z. B. als Drittlinientherapie) bestimmt ist.

15. Zusammensetzung zur Verwendung nach Anspruch 3 in Kombination mit einem Sulfonylharnstoff, die zur Verwendung bei der Verbesserung der Blutzuckereinstellung bei Patienten mit Diabetes Typ 2 mit unzureichender Blutzuckereinstellung trotz dualer Kombinationstherapie mit Metformin und einem Sulfonylharnstoff (z. B. als Drittlinientherapie) bestimmt ist.

16. Pharmazeutische Zusammensetzung zur Verwendung nach einem der Ansprüche 3, 12 bis 15, wobei der DPP-4-Hemmer in einer der folgenden Dosierungsstärken vorhanden ist:
2,5 mg, vorzugsweise zur oralen Verabreichung zweimal täglich; oder
5 mg, vorzugsweise zur oralen Verabreichung einmal täglich.

17. Pharmazeutische Zusammensetzung zur Verwendung nach einem der Ansprüche 3, 12 bis 16, wobei Metforminhydrochlorid in einer der folgenden Dosierungsstärken vorhanden ist:
500 mg, 850 mg oder 1000 mg, vorzugsweise in Form von Metformin mit sofortiger Freisetzung, vorzugsweise zur oralen Verabreichung zweimal täglich;
oder 500 mg, 750 mg, 1000 mg oder 1500 mg, vorzugsweise in Form von Metformin mit verzögerter Freisetzung, vorzugsweise zur oralen Verabreichung einmal täglich.

18. Pharmazeutische Zusammensetzung zur Verwendung nach einem der Ansprüche 3, 12 bis 17, wobei der DPP-4-Hemmer in einer Dosierungsstärke von 2,5 mg vorhanden ist und Metforminhydrochlorid in einer Dosierungsstärke von 500 mg, 850 mg oder 1000 mg vorhanden ist.

19. Pharmazeutische Zusammensetzung zur Verwendung nach einem der Ansprüche 3, 12 bis 18 zur oralen Verabreichung zweimal täglich an den Patienten.

20. Pharmazeutische Zusammensetzung zur Verwendung nach einem der Ansprüche 3, 12 bis 19, wobei L-Arginin in einer Menge von etwa 1 mg bis etwa 50 mg, vorzugsweise von etwa 1 mg bis etwa 25 mg, vorhanden ist.

21. Pharmazeutische Zusammensetzung zur Verwendung nach einem der Ansprüche 3, 12 bis 20, wobei der DPP-4-Hemmer und L-Arginin in einem Gewichtsverhältnis von etwa 1:20 bis etwa 10:1, oder ab etwa 1:15 bis etwa 10:1, bevorzugt ab etwa 1:10 bis etwa 10:1, vorhanden ist.

22. Pharmazeutische Zusammensetzung zur Verwendung nach einem der Ansprüche 3, 12 bis 21, wobei die Hilfsstoffe ausgewählt sind aus der Gruppe bestehend aus einem oder mehreren Füllstoffen, die D-Mannit, Maisstärke und vorgelatinierte Stärke beinhalten; einem Bindemittel, das Copovidon beinhaltet; einem Schmiermittel, das Magnesiumstearat beinhaltet; und einem Gleitmittel, das kolloidales wasserfreies Siliziumdioxid beinhaltet.

23. Pharmazeutische Zusammensetzung zur Verwendung nach einem der Ansprüche 3, 12 bis 22, umfassend Copovidon als Bindemittel.

24. Pharmazeutische Zusammensetzung zur Verwendung nach Anspruch 23, ferner umfassend eines oder mehrere der Folgenden: den Füllstoff Maisstärke, das Schmiermittel Magnesiumstearat und das Gleitmittel kolloidales wasserfreies Siliziumdioxid.

25. Pharmazeutische Zusammensetzung zur Verwendung nach einem der vorhergehenden Ansprüche in der Dosierungsform einer Tablette.

26. Pharmazeutische Zusammensetzung zur Verwendung nach Anspruch 25, wobei die Tablette ausgewählt ist aus einer Einschichttablette, einer Zweischichttablette, einer pressbeschichteten Tablette (was eine Tablette-in-Tablette und eine Bull's Eye-Tablette beinhaltet) und einer Tablette, die zur Arzneimittelbeladung mit einem Film überzogen ist.

27. Pharmazeutische Zusammensetzung zur Verwendung nach Anspruch 25 oder 26, wobei die Tablette einen Filmüberzug umfasst.

28. Pharmazeutische Zusammensetzung zur Verwendung nach Anspruch 27, wobei der Filmüberzug ein Filmüberzugsmittel, wie z. B. Hypromellose; einen Weichmacher, wie z. B. Propylenglycol; optional ein Gleitmittel, wie z. B. Talk, und optional ein oder mehrere Pigmente, wie z. B. Titandioxid, Eisenoxidrot und/oder Eisenoxidgelb, umfasst.

29. Pharmazeutische Zusammensetzung zur Verwendung nach einem der vorhergehenden Ansprüche, die eine Dosierungsform mit sofortiger Freisetzung ist, **dadurch gekennzeichnet, dass** in einem Auflösungstest nach 45 Minuten mindestens 75 % Massenanteil von jedem der Wirkstoffe gelöst wird.

30. Pharmazeutische Zusammensetzung zur Verwendung nach einem der vorhergehenden Ansprüche, wobei 1-[(4-Methyl-quinazolin-2-yl)methyl]-3-methyl-7-(2-butyn-1-yl)-8-(3-(R)-amino-piperidin-1-yl)-xanthin eine Teilchengrößenverteilung von X90 < 200 µm aufweist.

31. Pharmazeutische Zusammensetzung zur Verwendung nach Anspruch 3, umfassend die Wirkstoffe in einer Menge von:
(1) 2,5 mg 1-[(4-Methyl-quinazolin-2-yl)methyl]-3-methyl-7-(2-butyn-1-yl)-8-(3-(R)-amino-piperidin-1-yl)-xanthin-freie Base und 500 mg Metforminhydrochlorid; oder
(2) 2,5 mg 1-[(4-Methyl-quinazolin-2-yl)methyl]-3-methyl-7-(2-butyn-1-yl)-8-(3-(R)-amino-piperidin-1-yl)-xanthin-freie Base und 850 mg Metforminhydrochlorid; oder
(3) 2,5 mg 1-[(4-Methyl-quinazolin-2-yl)methyl]-3-methyl-7-(2-butyn-1-yl)-8-(3-(R)-amino-piperidin-1-yl)-xanthin-freie Base und 1000 mg Metforminhydrochlorid.

## Revendications

1. Composition pharmaceutique comprenant ou fabriquée à partir
d'un inhibiteur de la DPP-4 qui est la 1-[(4-méthyl-quinazolin-2-yl)méthyl]-3-méthyl-7-(2-butyn-1-yl)-8-(3-(R)-amino-pipéridin-1-yl)-xanthine,
de metformine,
d'un stabilisant qui est un acide aminé basique,
et d'un ou de plusieurs excipients pharmaceutiques ;
pour une utilisation dans le traitement et/ou la prévention (y compris le ralentissement de la progression et/ou le retard de l'apparition) de maladies métaboliques, en particulier le diabète sucré de type 2 et les affections qui y sont liées (par exemple, les complications diabétiques),
soit chez des patients diabétiques de type 2 non préalablement traités par un agent antihyperglycémiant,
soit chez des patients diabétiques de type 2 dont le contrôle glycémique est insuffisant malgré un traitement par un ou deux agents antihyperglycémiants conventionnels choisis parmi la metformine, les sulfonylurées, les thiazolidinediones (par exemple, la pioglitazone), les glinides, les inhibiteurs de l'alpha-glucosidase, le GLP-1 ou les analogues du GLP-1, et l'insuline ou les analogues de l'insuline ;
éventuellement en combinaison avec une ou plusieurs autres substances actives.

2. Composition pharmaceutique comprenant ou fabriquée à partir
d'un inhibiteur de la DPP-4 qui est la 1-[(4-méthyl-quinazolin-2-yl)méthyl]-3-méthyl-7-(2-butyn-1-yl)-8-(3-(R)-amino-pipéridin-1-yl)-xanthine,
de metformine, d'un stabilisant qui est un acide aminé basique,
et d'un ou de plusieurs excipients pharmaceutiques ;
pour une utilisation dans une ou plusieurs des méthodes suivantes :
- prévention, ralentissement de la progression, retard de l'apparition ou traitement d'un trouble ou d'une maladie métabolique, comme par exemple le diabète sucré de type 1, le diabète sucré de type 2, l'altération de la tolérance au glucose (IGT), l'altération de la glycémie à jeun (IFG), l'hyperglycémie, l'hyperglycémie postprandiale, le surpoids, l'obésité, la dyslipidémie, l'hyperlipidémie, l'hypercholestérolémie, l'hypertension, l'athérosclérose, le dysfonctionnement endothélial, l'ostéoporose, l'inflammation systémique chronique, la stéatose hépatique non alcoolique (NAFLD), la rétinopathie, la neuropathie, la néphropathie et/ou le syndrome métabolique ;
- amélioration du contrôle glycémique et/ou pour réduire la glycémie à jeun, la glycémie plasmatique postprandiale et/ou l'hémoglobine glycosylée HbA1c ;
- prévention, ralentissement, retard de l'apparition ou inversement de la progression d'une altération de la tolérance au glucose (IGT), d'une altération de la glycémie à jeun (IFG), de la résistance à l'insuline et/ou du syndrome métabolique au diabète de type 2 ;
- prévention, réduction du risque, ralentissement de la progression, retard de l'apparition ou traitement des complications du diabète sucré telles que les maladies micro- et macrovasculaires, telles que la néphropathie, la micro- ou macroalbuminurie, la protéinurie, la rétinopathie, la cataracte, la neuropathie, les troubles de l'apprentissage ou de la mémoire, les troubles neurodégénératifs ou cognitifs, les maladies cardio- ou cérébrovasculaires, l'ischémie tissulaire, le pied ou l'ulcère diabétique, l'athérosclérose, l'hypertension, le dysfonctionnement endothélial, l'infarctus du myocarde, le syndrome coronarien aigu, l'angine de poitrine instable, l'angine de poitrine stable, l'artériopathie périphérique oblitérante, la cardiomyopathie, l'insuffisance cardiaque, les troubles du rythme cardiaque, la resténose vasculaire et/ou l'accident vasculaire cérébral ;
- réduction du poids corporel ou prévention d'une augmentation du poids corporel ou facilitation d'une réduction du poids corporel ;
- prévention, ralentissement de la progression, retard de l'apparition ou traitement de la dégénérescence des cellules bêta pancréatiques et/ou le déclin de la fonctionnalité des cellules bêta pancréatiques et/ou pour améliorer et/ou restaurer la fonctionnalité des cellules bêta pancréatiques et/ou stimuler et/ou restaurer la fonctionnalité de la sécrétion d'insuline pancréatique ;
- prévention, ralentissement de la progression, retard de l'apparition ou traitement de la stéatose hépatique non alcoolique (NAFLD), y compris la stéatose hépatique, stéatohépatite non alcoolique (NASH) et/ou la fibrose hépatique ;
- prévention, ralentissement de la progression, retard de l'apparition ou traitement du diabète de type 2 avec échec primaire ou secondaire à une monothérapie ou polythérapie antihyperglycémiante conventionnelle (orale ou non orale) ;
- obtention d'une réduction de la dose de médicament antihyperglycémiant conventionnel nécessaire pour un effet thérapeutique adéquat ;
- réduction du risque d'effets indésirables associés aux médicaments antihyperglycémiants conventionnels ; et/ou
- maintien et/ou amélioration de la sensibilité à l'insuline et/ou pour traiter ou prévenir l'hyperinsulinémie et/ou la résistance à l'insuline ;
soit chez un patient diabétique de type 2 non préalablement traité par un agent antihyperglycémiant soit chez un patient diabétique de type 2 dont le contrôle glycémique est insuffisant malgré un traitement par un ou deux agents antihyperglycémiants conventionnels choisis parmi la metformine, les sulfonylurées, les thiazolidinediones, les glinides, les inhibiteurs de l'alpha-glucosidase, le GLP-1 ou les analogues du GLP-1, et l'insuline ou les analogues de l'insuline ;
éventuellement en combinaison avec une ou plusieurs autres substances actives.

3. Composition pharmaceutique pour une utilisation selon la revendication 1 ou 2, qui est une composition pharmaceutique solide (par exemple un comprimé oral) comprenant ou fabriquée à partir
de la 1-[(4-méthyl-quinazolin-2-yl)méthyl]-3-méthyl-7-(2-butyn-1-yl)-8-(3-(R)-amino-pipéridin-1-yl)-xanthine,
du chlorhydrate de metformine,
de la L-arginine (en particulier comme stabilisant),
et d'une ou de plusieurs charges, d'un ou de plusieurs liants, d'un ou de plusieurs agents de glissement et/ou d'un ou de plusieurs lubrifiants.

4. Composition pharmaceutique pour une utilisation selon la revendication 1, 2 ou 3, dans laquelle les patients diabétiques de type 2 n'ont pas été préalablement traités avec un agent antihyperglycémiant (par exemple des patients naïfs de traitement).

5. Composition pharmaceutique pour une utilisation selon la revendication 1, 2 ou 3, dans laquelle les patients diabétiques de type 2 ont un contrôle glycémique insuffisant sur la metformine, avec ou sans une thiazolidinedione (par exemple, la pioglitazone) ou une sulfonylurée.

6. Composition pharmaceutique pour une utilisation selon la revendication 1, 2 ou 3, dans laquelle les patients diabétiques de type 2 ont un contrôle glycémique insuffisant malgré une monothérapie avec la metformine.

7. Composition pharmaceutique pour une utilisation selon la revendication 1, 2 ou 3, en combinaison avec une thiazolidinedione (par exemple, la pioglitazone), dans laquelle les patients diabétiques de type 2 ont un contrôle glycémique insuffisant malgré une bithérapie avec la metformine et une thiazolidinedione (par exemple, la pioglitazone).

8. Composition pharmaceutique pour une utilisation selon la revendication 1, 2 ou 3, en combinaison avec une sulfonylurée, dans laquelle les patients diabétiques de type 2 ont un contrôle glycémique insuffisant malgré une bithérapie avec la metformine et une sulfonylurée.

9. Composition pharmaceutique pour une utilisation selon l'une quelconque des revendications 1 à 8, dans laquelle la maladie métabolique est choisie parmi le diabète sucré de type 1, le diabète sucré de type 2, l'altération de la tolérance au glucose (IGT), l'altération de la glycémie à jeun (IFG), l'hyperglycémie, l'hyperglycémie postprandiale, le surpoids, l'obésité, la dyslipidémie, l'hyperlipidémie, l'hypercholestérolémie, l'hypertension, l'athérosclérose, le dysfonctionnement endothélial, l'ostéoporose, l'inflammation systémique chronique, la stéatose hépatique non alcoolique (NAFLD), la rétinopathie, la neuropathie, la néphropathie et le syndrome métabolique.

10. Composition pharmaceutique pour une utilisation selon l'une quelconque des revendications 1 à 8, dans laquelle la complication diabétique est choisie parmi les maladies micro- et macrovasculaires, telles que la néphropathie, la micro- ou macroalbuminurie, la protéinurie, la rétinopathie, la cataracte, la neuropathie, les troubles de l'apprentissage ou de la mémoire, les troubles neurodégénératifs ou cognitifs, les maladies cardio- ou cérébrovasculaires, l'ischémie tissulaire, le pied ou l'ulcère diabétique, l'athérosclérose, l'hypertension, le dysfonctionnement endothélial, l'infarctus du myocarde, le syndrome coronarien aigu, l'angine de poitrine instable, l'angine de poitrine stable, l'artériopathie périphérique oblitérante, cardiomyopathie, l'insuffisance cardiaque, les troubles du rythme cardiaque, la resténose vasculaire et l'accident vasculaire cérébral.

11. Composition pharmaceutique pour une utilisation selon l'une quelconque des revendications 1 à 8, dans laquelle la maladie métabolique est le diabète sucré de type 2.

12. Composition pour une utilisation selon la revendication 3, qui est destinée à être utilisée pour améliorer le contrôle glycémique chez des patients diabétiques de type 2 qui n'ont pas été préalablement traités avec un agent antihyperglycémique (par exemple, comme thérapie de première ligne) .

13. Composition pour une utilisation selon la revendication 3, qui est destinée à être utilisée pour améliorer le contrôle glycémique chez des patients diabétiques de type 2 ayant un contrôle glycémique insuffisant malgré une monothérapie avec la metformine (par exemple, comme thérapie de deuxième ligne).

14. Composition pour une utilisation selon la revendication 3 en combinaison avec une thiazolidinedione (par exemple, la pioglitazone), qui est destinée à être utilisée pour améliorer le contrôle glycémique chez les patients diabétiques de type 2 ayant un contrôle glycémique insuffisant malgré une bithérapie avec la metformine et une thiazolidinedione (par exemple, comme thérapie de troisième ligne).

15. Composition pour une utilisation selon la revendication 3, en combinaison avec une sulphonylurée, qui est destinée à être utilisée pour améliorer le contrôle glycémique chez des patients diabétiques de type 2 ayant un contrôle glycémique insuffisant malgré une monothérapie avec la metformine et une sulphonylurée (par exemple, comme thérapie de troisième ligne).

16. Composition pharmaceutique pour une utilisation selon l'une quelconque des revendications 3, 12 à 15, dans laquelle l'inhibiteur de la DPP-4 est présent en une dose
de 2,5 mg, de préférence pour une administration orale deux fois par jour ; ou
de 5 mg, de préférence pour une administration orale une fois par jour.

17. Composition pharmaceutique pour une utilisation selon l'une quelconque des revendications 3, 12 à 16, dans laquelle le chlorhydrate de metformine est présent en une dose
de 500 mg, 850 mg ou 1 000 mg, de préférence sous forme de metformine à libération immédiate, de préférence pour une administration orale deux fois par jour ;
ou de 500 mg, 750 mg, 1 000 mg ou 1 500 mg, de préférence sous forme de metformine à libération prolongée, de préférence pour une administration orale une fois par jour.

18. Composition pharmaceutique pour une utilisation selon l'une quelconque des revendications 3, 12 à 17, dans laquelle l'inhibiteur de la DPP-4 est présent en une dose de 2,5 mg et le chlorhydrate de metformine est présent en une dose de 500 mg, 850 mg ou 1 000 mg.

19. Composition pharmaceutique pour une utilisation selon l'une quelconque des revendications 3, 12 à 18, qui est destinée à une administration orale deux fois par jour au patient.

20. Composition pharmaceutique pour une utilisation selon l'une quelconque des revendications 3, 12 à 19, dans laquelle la L-arginine est présente d'environ 1 mg à environ 50 mg, de préférence d'environ 1 mg à environ 25 mg.

21. Composition pharmaceutique pour une utilisation selon l'une quelconque des revendications 3, 12 à 20, dans laquelle l'inhibiteur de la DPP-4 et la L-arginine sont présents dans un rapport pondéral d'environ 1:20 à environ 10:1, ou d'environ 1:15 à environ 10:1, de préférence d'environ 1:10 à environ 10:1.

22. Composition pharmaceutique pour une utilisation selon l'une quelconque des revendications 3, 12 à 21, dans laquelle les excipients sont choisis dans le groupe constitué d'une ou de plusieurs charges comprenant le D-mannitol, l'amidon de maïs et l'amidon prégélatinisé ; un liant comportant de la copovidone ; un lubrifiant comportant du stéarate de magnésium ; et un agent de glissement comportant de la silice colloïdale anhydre.

23. Composition pharmaceutique pour une utilisation selon l'une quelconque des revendications 3, 12 à 22 comprenant de la copovidone comme liant.

24. Composition pharmaceutique pour une utilisation selon la revendication 23, comprenant en outre un ou plusieurs des éléments suivants : la charge d'amidon de maïs, le lubrifiant de stéarate de magnésium et l'agent de glissement de silice colloïdale anhydre.

25. Composition pharmaceutique pour une utilisation selon l'une quelconque des revendications précédentes sous la forme posologique d'un comprimé.

26. Composition pharmaceutique pour une utilisation selon la revendication 25, dans laquelle le comprimé est choisi parmi un comprimé monocouche, un comprimé bicouche, un comprimé enrobé par pression (y compris un comprimé dans un comprimé et un comprimé à noyau), et un comprimé pelliculé pour le chargement du médicament.

27. Composition pharmaceutique pour une utilisation selon la revendication 25 ou 26, dans laquelle le comprimé comprend un pelliculage.

28. Composition pharmaceutique pour une utilisation selon la revendication 27, dans laquelle le pelliculage comprend un agent de pelliculage, tel que, par exemple, l'hypromellose ; un plastifiant, tel que par exemple le propylène glycol ; éventuellement un agent de glissement, tel que par exemple le talc, et éventuellement un ou plusieurs pigments, tels que par exemple le dioxyde de titane, l'oxyde de fer rouge et/ou l'oxyde de fer jaune.

29. Composition pharmaceutique pour une utilisation selon l'une quelconque des revendications précédentes, qui est une forme posologique à libération immédiate, **caractérisée en ce que** dans un test de dissolution après 45 minutes au moins 75 % en poids de chacun des ingrédients actifs sont dissous.

30. Composition pharmaceutique pour une utilisation selon l'une quelconque des revendications précédentes, dans laquelle la 1-[(4-méthyl-quinazolin-2-yl)méthyl]-3-méthyl-7-(2-butyn-1-yl)-8-(3-(R)-amino-pipéridin-1-yl)-xanthine a une distribution granulométrique de X90 < 200 µm.

31. Composition pharmaceutique pour une utilisation selon la revendication 3 comprenant les ingrédients actifs en une quantité de :
(1) 2,5 mg de base libre de 1-[(4-méthyl-quinazolin-2-yl)méthyl]-3-méthyl-7-(2-butyn-1-yl)-8-(3-(R)-amino-pipéridin-1-yl)-xanthine et 500 mg de chlorhydrate de metformine ; ou
(2) 2,5 mg de base libre de 1-[(4-méthyl-quinazolin-2-yl)méthyl]-3-méthyl-7-(2-butyn-1-yl)-8-(3-(R)-amino-pipéridin-1-yl)-xanthine, et 850 mg de chlorhydrate de metformine ; ou
(3) 2,5 mg de base libre de 1-[(4-méthyl-quinazolin-2-yl)méthyl]-3-méthyl-7-(2-butyn-1-yl)-8-(3-(R)-amino-pipéridin-1-yl)-xanthine, et 1 000 mg de chlorhydrate de metformine.
